# EUROPEAN PATENT APPLICATION

(11) **EP 4 763 984 A1**
(43) Date of publication of application: **24.06.2026**
(21) Application number: 24223117.3
(22) Date of filing: 23.12.2024
(51) Int. Cl.: C12N 15/11, C12N 15/113

(54) **RNA PRODUCTS AND METHODS OF PRODUCTION**

(71) Applicant: 4basebio UK Ltd, Cambridge CB24 5QE (GB)
(72) Inventor: BOUCHAREB, Amine, Cambridge, CB24 5QE (GB); DHIR, Ashish, Cambridge, CB24 5QE (GB); WALKER, Amy, Cambridge, CB24 5QE (GB); LANCKRIET, Heikki, Cambridge, CB24 5QE (GB); CHRISTIAN, Swar, Cambridge, CB24 5QE (GB)
(74) Representative: Boult Wade Tennant LLP

(57) **Abstract**

The invention relates to methods for producing an RNA product (e.g. a guide RNA or prime editing guide RNA (pegRNA)) comprising ligating at least three RNA molecules by splint ligation. The at least three RNA molecules comprise: a first RNA molecule that is a chemically synthesized RNA molecule, a second RNA molecule that is an in vitro transcription (IVT) produced RNA molecule, and a third RNA molecule that is a chemically synthesized RNA molecule. The invention also relates to RNA products produced by the methods and the uses of such products.

## Description

### TECHNICAL FIELD

The invention relates to methods for producing an RNA product (e.g. a guide RNA or prime editing guide RNA (pegRNA)) comprising ligating at least three RNA molecules by splint ligation. The at least three RNA molecules comprise: a first RNA molecule that is a chemically synthesized RNA molecule, a second RNA molecule that is an in vitro transcription (IVT) produced RNA molecule, and a third RNA molecule that is a chemically synthesized RNA molecule. The invention also relates to RNA products produced by the methods and the uses of such products.

### BACKGROUND

There are a wide range of uses for RNA molecules including research applications, diagnostic applications, RNA therapeutics (such as gene therapies e.g. therapies based on ASO, siRNA and CRISPR-Cas) and mRNA-based vaccines (e.g. mRNA-based COVID-19 vaccines) (Pfeifer et al., 2023, npj Systems Biology and Applications (2023) 9:60, https://doi.org/10.1038/s41540-023-00323-3).

RNA molecules may be obtained in three ways: purification from biological sources; chemical synthesis using a solid-phase method; or enzymatic synthesis by in vitro transcription (IVT). All of these approaches have limitations. Purification from cells is suitable for complex or large and highly abundant RNAs; however, it is not ideal for the production of RNA therapeutics (or other applications for which high purity RNA molecules are required). Chemical synthesis allows the generation of high purity RNA molecules with modifications, but its major drawback is the size limitation of about 100 nucleotides. For larger RNA molecules, RNA is typically generated by in vitro transcription (Flemmich et al., 2024, Angew. Chem. Int. Ed. 2024, 63, e202403063). Methods have also emerged to address the production of specific RNAs. For example, Lei et al., have developed such a method that combines two RNA molecules to generate prime editing guide RNA (pegRNA) (Nat Biotechnol (2024). https://doi.org/10.1038/s41587-024-02394-x).

Notwithstanding the availability of this range of techniques, there is a need for methods that enable the production of large and chemically modified RNAs with a high yield and purity. There is also a need for methods that provide these benefits at a reasonable cost.

### DESCRIPTION

The inventors have developed methods for producing RNA products (including large and chemically modified RNA products). The methods can be used to produce a high yield of an RNA product and the RNA product may have a high purity. Furthermore, the methods enable the production of more stable RNA molecules that are resistant to degradation (e.g. degradation by exonucleases). The methods also enable the production of RNA products in a cost-effective manner for the applications for which they are required.

The invention provides methods for producing an RNA product (e.g. a guide RNA or prime editing guide RNA (pegRNA)) comprising ligating at least three RNA molecules by splint ligation. The at least three RNA molecules comprise: a first RNA molecule that is a chemically synthesized RNA molecule, a second RNA molecule that is an in vitro transcription (IVT) produced RNA molecule, and a third RNA molecule that is a chemically synthesized RNA molecule. The approach means that RNA products can be designed in a rational way such that: IVT may be used to synthesize a large RNA molecule (herein the second RNA molecule) that does not require chemical modification; and chemical synthesis may be used to synthesize smaller RNA molecules (herein the first and third RNA molecules) that do require chemical modification (e.g. to increase the stability of the RNA product).

A method for producing an RNA product, wherein the method comprises:
a) providing a first RNA molecule, a second RNA molecule and a third RNA molecule; and
b) ligating the 3' end of the first RNA molecule to the 5' end of the second RNA molecule and ligating the 3' end of the second RNA molecule to the 5' end of the third RNA molecule, wherein the ligating is performed by splint ligation;
wherein the first RNA molecule is a chemically synthesized RNA molecule, the second RNA molecule is an in vitro transcription (IVT) produced RNA molecule, and the third RNA molecule is a chemically synthesized RNA molecule.

The first RNA molecule may comprise a nuclease-resistant nucleotide and the third RNA molecule may comprise a nuclease-resistant nucleotide.

The RNA product may be a linear RNA product.

The RNA product may comprise a long RNA, a long non-coding RNA (IncRNA), a non-coding RNA (ncRNA), a guide RNA (gRNA), a single guide RNA (sgRNA), a prime editing guide RNA (pegRNA) or an mRNA (e.g. an mRNA encoding a pathogen-specific antigen or an mRNA encoding a neoantigen). The gRNA may comprise an aptamer sequence (e.g. MS2 aptamer sequence).

The RNA product may comprise an RNA cassette. The RNA cassette may comprise a long RNA, a long non-coding RNA (IncRNA), a non-coding RNA (ncRNA), a guide RNA (gRNA), a prime editing guide RNA (pegRNA) or an mRNA (e.g. an mRNA encoding a pathogen-specific antigen or an mRNA encoding a neoantigen). The gRNA may comprise an aptamer sequence (e.g. MS2 aptamer sequence).

The RNA product may be at least 50 nucleotides, at least 75 nucleotides, at least 100 nucleotides, at least 105 nucleotides, at least 110 nucleotides, at least 115 nucleotides, at least 120 nucleotides, at least 125 nucleotides, at least 135 nucleotides, at least 145 nucleotides, at least 150 nucleotides, at least 155 nucleotides, at least 160 nucleotides, at least 165 nucleotides, at least 170 nucleotides, at least 175 nucleotides, at least 180 nucleotides, at least 185 nucleotides, at least 190 nucleotides, at least 195 nucleotides, at least 200 nucleotides, at least 250 nucleotides, at least 300 nucleotides, at least 400 nucleotides, at least 500 nucleotides, at least 750 nucleotides, at least 1000 nucleotides, at least 1500 nucleotides, at least 2000 nucleotides, at least 3000 nucleotides, at least 4000 nucleotides, at least 5000 nucleotides, at least 6000 nucleotides, at least 7000 nucleotides, at least 8000 nucleotides or at least 9000 nucleotides. Preferably, the RNA product is at least 135 nucleotides.

The RNA product may be a protected linear RNA. The RNA product may comprise two or more nuclease resistant nucleotides (i.e. nucleotides resistant to exonuclease digestion). The RNA product may be resistant to 5'-3' exonuclease digestion and/or 3'-5' exonuclease digestion. The RNA product may comprise at least 1 nuclease resistant nucleotide 5' of the RNA cassette and at least 1 nuclease resistant nucleotide 3' of the RNA cassette. The RNA product may comprise at least 2 nuclease resistant nucleotides 5' of the RNA cassette and at least 2 nuclease resistant nucleotides 3' of the RNA cassette. The RNA product may comprise at least 3 nuclease resistant nucleotides 5' of the RNA cassette and at least 3 nuclease resistant nucleotides 3' of the RNA cassette. The RNA product may comprise at least 4 nuclease resistant nucleotides 5' of the RNA cassette and at least 4 nuclease resistant nucleotides 3' of the RNA cassette. The RNA product may comprise at least 5 nuclease resistant nucleotides 5' of the RNA cassette and at least 5 nuclease resistant nucleotides 3' of the RNA cassette. Preferably, the RNA product comprises at least 2 nuclease resistant nucleotides 5' of the RNA cassette and at least 2 nuclease resistant nucleotides 3' of the RNA cassette.

The invention provides a method for producing an RNA product, wherein the method comprises:
a) providing a first RNA molecule, a second RNA molecule, and a third RNA molecule,; and
b) ligating the 3' end of the first RNA molecule to the 5' end of the second RNA molecule and ligating the 3' end of the second RNA molecule to the 5' end of the third RNA molecule, wherein the ligating is performed by splint ligation;
wherein the first RNA molecule is a chemically synthesized RNA molecule, the second RNA molecule is an in vitro transcription (IVT) produced RNA molecule, and the third RNA molecule is a chemically synthesized RNA molecule.

The invention provides a method for producing a guide RNA, wherein the method comprises:
a) providing a first RNA molecule, a second RNA molecule, and a third RNA molecule, wherein the first RNA molecule comprises a nuclease-resistant nucleotide and the third RNA molecule comprises a nuclease-resistant nucleotide; and
b) ligating the 3' end of the first RNA molecule to the 5' end of the second RNA molecule and ligating the 3' end of the second RNA molecule to the 5' end of the third RNA molecule, wherein the ligation is performed by splint ligation;

wherein the guide RNA comprises, in the 5'-3' direction, a spacer sequence and a scaffold region; and
wherein the first RNA molecule is a chemically synthesized RNA molecule, the second RNA molecule is an in vitro transcription (IVT) produced RNA molecule, and the third RNA molecule is a chemically synthesized RNA molecule.

The first RNA may comprise at least a portion of the spacer sequence and/or the second RNA molecule may comprise at least a portion of the scaffold region.

The first RNA may comprise at least a portion of the scaffold region.

The first RNA may comprise at least a portion of the spacer sequence and at least a portion of the scaffold region.

The spacer region (or targeting sequence) may anneal to a genomic target sequence.

The scaffold region is capable of binding a CRISPR-associated endonuclease e.g. Cas9 or Cpf1. The scaffold region may comprise a tracrRNA. The scaffold region may comprise a tracrRNA and a linker region connecting the tracrRNA to the crRNA.

The invention provides a method for producing a guide RNA, wherein the method comprises:
a) providing a first RNA molecule comprising a spacer sequence and at least a portion of a scaffold region, a second RNA molecule comprising at least a portion of a scaffold region, and a third RNA molecule comprising at least a portion of a scaffold region, wherein the first RNA molecule comprises a nuclease-resistant nucleotide and the third RNA molecule comprises a nuclease-resistant nucleotide; and
b) ligating the 3' end of the first RNA molecule to the 5' end of the second RNA molecule and ligating the 3' end of the second RNA molecule to the 5' end of the third RNA molecule, wherein the ligation is performed by splint ligation;

wherein the guide RNA comprises, in the 5'-3' direction, a spacer sequence and a scaffold region; and
wherein the first RNA molecule is a chemically synthesized RNA molecule, the second RNA molecule is an in vitro transcription (IVT) produced RNA molecule, and the third RNA molecule is a chemically synthesized RNA molecule.

The guide RNA may be a prime editing guide RNA (pegRNA) and wherein the pegRNA comprises, in the 5'-3' direction, a spacer sequence, a scaffold region, a reverse transcription template (RTT) and a primer binding site (PBS).

The invention provides a method of producing a prime editing guide RNA (pegRNA), wherein the method comprises:
a) providing a first RNA molecule, a second RNA molecule comprising a scaffold region, and a third RNA molecule, optionally wherein the first RNA molecule comprises a protected nucleotide and the third RNA molecule comprises a protected nucleotide; and
b) ligating the 3' end of the first RNA molecule to the 5' end of the second RNA molecule and ligating the 3' end of the second RNA molecule to the 5' end of the third RNA molecule, wherein the ligating is performed by splint ligation;

wherein the pegRNA comprises, in the 5'-3' direction, a spacer sequence, a scaffold region, a reverse transcription template (RTT) and a primer binding site (PBS); and
wherein the first RNA molecule is a chemically synthesized RNA molecule, the second RNA molecule is an in vitro transcription (IVT) produced RNA molecule, and the third RNA molecule is a chemically synthesized RNA molecule.

The first RNA molecule may comprise at least a portion of the scaffold region.

The second RNA molecule may comprise the reverse transcription template (RTT). The third RNA molecule may comprise the reverse transcription template (RTT). The second RNA molecule may comprise a portion of the reverse transcription template (RTT) and the third RNA molecule may comprise a portion of the reverse transcription template (RTT).

The third RNA molecule may comprise the primer binding site (PBS).

The invention provides a method of producing a prime editing guide RNA (pegRNA), wherein the method comprises:
a) providing a first RNA molecule comprising a spacer sequence, a second RNA molecule comprising at least a portion of a scaffold region, and a third RNA molecule comprising a primer binding site (PBS), optionally wherein the first RNA molecule comprises a protected nucleotide and the third RNA molecule comprises a protected nucleotide; and
b) ligating the 3' end of the first RNA molecule to the 5' end of the second RNA molecule and ligating the 3' end of the second RNA molecule to the 5' end of the third RNA molecule, wherein the ligating is performed by splint ligation;

wherein the pegRNA comprises, in the 5'-3' direction, a spacer sequence, a scaffold region, a reverse transcription template (RTT) and a primer binding site (PBS); and
wherein the first RNA molecule is a chemically synthesized RNA molecule, the second RNA molecule is an in vitro transcription (IVT) produced RNA molecule, and the third RNA molecule is a chemically synthesized RNA molecule.

The first RNA molecule may comprise at least a portion of the scaffold region.

The second RNA molecule may comprise the reverse transcription template (RTT). The third RNA molecule may comprise the reverse transcription template (RTT). The second RNA molecule may comprise a portion of the reverse transcription template (RTT) and the third RNA molecule may comprise a portion of the reverse transcription template (RTT).

The invention provides a method of producing a prime editing guide RNA (pegRNA), wherein the method comprises:
a) providing a first RNA molecule comprising a spacer sequence, a second RNA molecule comprising at least a portion of a scaffold region and at least a portion of a reverse transcription template (RTT), and a third RNA molecule comprising at least a portion of a reverse transcription template (RTT) and a primer binding site (PBS), optionally wherein the first RNA molecule comprises a protected nucleotide and the third RNA molecule comprises a protected nucleotide; and
b) ligating the 3' end of the first RNA molecule to the 5' end of the second RNA molecule and ligating the 3' end of the second RNA molecule to the 5' end of the third RNA molecule, wherein the ligating is performed by splint ligation;

wherein the pegRNA comprises, in the 5'-3' direction, a spacer sequence, a scaffold region, a reverse transcription template (RTT) and a primer binding site (PBS); and
wherein the first RNA molecule is a chemically synthesized RNA molecule, the second RNA molecule is an in vitro transcription (IVT) produced RNA molecule, and the third RNA molecule is a chemically synthesized RNA molecule.

The first RNA molecule may comprise at least a portion of the scaffold region.

The invention provides a method of producing a prime editing guide RNA (pegRNA), wherein the method comprises:
a) providing a first RNA molecule comprising a spacer sequence, a second RNA molecule comprising at least a portion of a scaffold region, and a third RNA molecule comprising a reverse transcription template (RTT) and a primer binding site (PBS), optionally wherein the first RNA molecule comprises a protected nucleotide and the third RNA molecule comprises a protected nucleotide; and
b) ligating the 3' end of the first RNA molecule to the 5' end of the second RNA molecule and ligating the 3' end of the second RNA molecule to the 5' end of the third RNA molecule, wherein the ligating is performed by splint ligation;

wherein the pegRNA comprises, in the 5'-3' direction, a spacer sequence, a scaffold region, a reverse transcription template (RTT) and a primer binding site (PBS); and
wherein the first RNA molecule is a chemically synthesized RNA molecule, the second RNA molecule is an in vitro transcription (IVT) produced RNA molecule, and the third RNA molecule is a chemically synthesized RNA molecule.

The first RNA molecule may comprise at least a portion of the scaffold region.

In the methods, step (a) may comprise producing the first RNA molecule and the third RNA molecule by chemical synthesis. In the methods, step (a) may comprise producing the second RNA molecule by in vitro transcription (IVT). In the methods, step (a) may comprise: producing the first RNA molecule and the third RNA molecule by chemical synthesis; and producing the second RNA molecule by in vitro transcription (IVT).

The chemical synthesis may comprise solid-phase synthesis (e.g. solid-phase synthesis using phosphoramidite chemistry).

The step of producing the second RNA by in vitro transcription may comprise producing an adaptor-ligated DNA product as a template for in vitro transcription (IVT). Methods for producing an adaptor-ligated DNA product are described herein.

The step of producing the second RNA by in vitro transcription may comprise producing a PCR product as a template for in vitro transcription (IVT).

The step of producing the second RNA by in vitro transcription may comprise using chemical synthesis to produce the template for in vitro transcription (IVT). The chemical synthesis may comprise solid-phase synthesis (e.g. solid-phase synthesis using phosphoramidite chemistry).

In the methods, the second RNA molecule may be produced by IVT in a reaction that comprises GMP. The reaction may comprise a higher concentration of GMP than GTP. The reaction may comprise a ratio of at least 3:1, at least 4:1 or at least 5:1 (GMP:GTP)

In the methods, IVT may be performed using at least 3 mM, at least 4 mM, at least 5 mM, at least 6 mM, at least 7 mM, at least 8 mM, at least 9 mM, at least 10 mM, at least 15 mM or at least 20 mM dNTPs. Preferably, IVT is performed using at least 5 mM NTPs.

In the methods, IVT may be performed using 3-20 mM, 4-15 mM or 5-10 mM NTPs.

In the methods, IVT may be performed using a T3 RNA polymerase, a T7 RNA polymerase, an SP6 RNA polymerase, a Therminator polymerase, an E. coli RNA polymerase (Core Enzyme), E. coli RNA polymerase (Holoenzyme) and/or VSW-3 RNA polymerase.

In the methods, the second RNA molecule may have a 5'-terminal GTP and step (a) may comprise treating the second RNA molecule with a phosphatase to cleave phosphate groups from the 5'-terminal GTP to produce a second RNA molecule with a 5'-terminal GMP. The phosphatase may be E. *coli* 5'-RNA pyrophosphohydrolase (RppH). The phosphatase may be an alkaline phosphatase, wherein the alkaline phosphatase removes all phosphate groups from the 5' GTP, and the step of treating the second RNA with a phosphatase may be followed by treatment with a T4 polynucleotide kinase to phosphorylate guanosine and generate a 5'-terminal GMP.

In the methods, step (b) may comprise: annealing the first, second and third RNA molecules to a splint DNA; and ligating the 3' end of the first RNA molecule to the 5' end of the second RNA molecule and ligating the 3' end of the second RNA molecule to the 5' end of the third RNA molecule. Preferably, the splint DNA is a single splint DNA molecule.

Preferably, the splint DNA is a single stranded DNA molecule.

The splint DNA may be two splint DNA molecules. The first RNA molecule may be annealed (completely or partially) to a first splint DNA molecule, the second RNA molecule may be partially annealed to the first splint DNA molecule and partially annealed to a second splint DNA molecule, and the third RNA molecule may be annealed (completely or partially) to the second splint DNA molecule.

The splint DNA may be a protected DNA comprising a single-stranded DNA (ssDNA) cassette, wherein the protected DNA comprises x nuclease-resistant nucleotides at the 5' end of the ssDNA cassette or 5' of the ssDNA cassette, and y nuclease-resistant nucleotides at the 3'-end of the ssDNA cassette or 3' of the ssDNA cassette, wherein x is at least 1 and y is at least 1.

The splint DNA may not comprise any nuclease-resistant nucleotides.

The splint DNA may be a chemically synthesized DNA molecule. The method may comprise producing the splint DNA by chemical synthesis. The chemical synthesis may comprise solid-phase synthesis (e.g. solid-phase synthesis using phosphoramidite chemistry).

In the methods, the template for in vitro transcription (IVT) and/or the splint DNA may be chemically synthesized DNA molecules. The chemical synthesis may comprise solid-phase synthesis (e.g. solid-phase synthesis using phosphoramidite chemistry).

In the methods, the step of producing the second RNA by in vitro transcription may comprise using chemical synthesis to produce the template for in vitro transcription (IVT) and using chemical synthesis to produce the splint DNA. The chemical synthesis may comprise solid-phase synthesis (e.g. solid-phase synthesis using phosphoramidite chemistry).

The splint DNA may be a protected DNA comprising a single-stranded DNA (ssDNA) cassette, and wherein the method comprises:
(a) providing a partially protected double-stranded DNA (dsDNA) comprising a first strand and a second strand, wherein the first strand of the partially protected dsDNA comprises a cassette, x nuclease-resistant nucleotides at the 5' end of the cassette or 5' of the cassette, and y nuclease-resistant nucleotides at the 3'-end of the cassette or 3' of the cassette, wherein x is at least 1 and y is at least 1; and
(b) digesting the second strand of the partially protected dsDNA with an exonuclease thereby generating the protected DNA.

The splint DNA may comprise a sequence comprising regions complementary to the scaffold region and spacer sequence. The splint DNA may comprise a sequence comprising regions complementary to the primer binding site (PBS), reverse transcription template (RTT), scaffold region and/or spacer sequence. The splint DNA may anneal to at least 90%, at least 95%, at least 96%, at least 97%, at least 98% or at least 99% of the RNA product sequence (e.g. guide RNA or pegRNA sequence).

The method may further comprise digesting the splint DNA using DNase. The DNase may be DNase I and/or Duplex DNase. Additionally or alternatively, the step of digesting the splint DNA may be performed using one or more exonucleases e.g. Exolll.

The method may further comprise digesting the unligated RNA molecules using one or more RNA exonucleases. The RNA exonuclease(s) may be a 5'-3' RNA exonuclease and/or a 3'-5' RNA exonuclease. The one or more RNA exonucleases may be selected from XRN-I (5' to 3'), Terminator 5'-Phosphate-Dependent Exonuclease (5' to 3'), Exonuclease T (3' to 5'), Exolll (3' to 5') and/or RNase R (3' to 5').

In the methods, the step of splint ligation may be performed using T3 DNA ligase, Thermostable 5' App DNA/RNA Ligase, SplintR^{®} Ligase, T4 RNA ligase and/or RtcB Ligase. Preferably the step of splint ligation is performed using T4 RNA ligase. The T4 RNA ligase may be T4 RNA ligase 1 or T4 RNA ligase 2.

In the methods, the first RNA molecule may comprise a protected nucleotide 5' of the spacer sequence and/or in a 5' end region. The third RNA molecule may comprises a nuclease-resistant nucleotide 3' of the primer binding site (e.g. if the RNA product is a pegRNA) and/or in a 3' end region.

The method may be a cell-free method. The method may be an in vitro method. The method may be a cell-free, in vitro method.

The invention provides a guide RNA for use in a method of treating a disease, wherein the guide RNA is produced according to the method of any one of claims 1-14 and wherein the guide RNA is used to either (i) edit a target gene by gene editing and thereby treat the disease or (ii) activate the transcription of endogenous gene and thereby treat the disease.

The invention provides a method of treating a disease by gene editing comprising: a) producing a guide RNA according to any of the methods described herein; and b) using the guide RNA to either (i) edit a target gene by gene editing and thereby treat the disease or (ii) activate the transcription of endogenous gene and thereby treat the disease.

The invention provides a pegRNA for use in a method of treating a disease, wherein the pegRNA is produced according to any of the methods described herein and wherein the pegRNA is used to either (i) edit a target gene by prime editing and thereby treat the disease or (ii) activate the transcription of endogenous gene and thereby treat the disease.

The invention provides a method of treating a disease by prime editing comprising: a) producing a pegRNA according to any of the methods described herein; and b) using the pegRNA to either (i) edit a target gene by prime editing and thereby treat the disease or (ii) activate the transcription of endogenous gene and thereby treat the disease.

The invention provides a method of producing an RNA vaccine, wherein the method comprises:
(a) producing an RNA product using any of the methods described herein, wherein the RNA product comprises an mRNA encoding a pathogen-specific antigen; and
(b) formulating the RNA product to produce the RNA vaccine.

The invention provides a method of producing an RNA vaccine, wherein the method comprises:
(a) producing an RNA product using any of the methods described herein, wherein the RNA product comprises an mRNA encoding a neoantigen; and
(b) formulating the RNA product to produce the RNA vaccine.

### 1. Properties of the first, second and third RNA molecules

The first RNA molecule and/or the third RNA molecule may (each) comprise at least 5 nucleotides, at least 10 nucleotides, at least 15 nucleotides, at least 20 nucleotides, at least 25 nucleotides, at least 30 nucleotides, at least 35 nucleotides, at least 40 nucleotides, at least 50 nucleotides, at least 60 nucleotides, at least 70 nucleotides, at least 80 nucleotides, at least 90 nucleotides, at least 100 nucleotides or at least 110 nucleotides. Preferably, the first RNA molecule and/or the third RNA molecule (each) comprise at least 10 nucleotides.

The first RNA molecule and/or the third RNA molecule may (each) comprise less than 100 nucleotides, less than 90 nucleotides, less than 80 nucleotides, less than 70 nucleotides, less than 60 nucleotides, less than 50 nucleotides, less than 45 nucleotides, less than 40 nucleotides, less than 35 nucleotides, less than 30 nucleotides, less than 25 nucleotides, less than 20 nucleotides, less than 15 nucleotides, less than 10 nucleotides or less than 5 nucleotides. Preferably, the first RNA molecule and/or the third RNA molecule may (each) comprise less than 25 nucleotides.

The first RNA molecule and/or the third RNA molecule may (each) comprise 5-120 nucleotides, 10-110 nucleotides, 15-100 nucleotides, 20-90 nucleotides, 30-80 nucleotides, 40-70 nucleotides or 50-60 nucleotides. Preferably, the first RNA molecule and/or the third RNA molecule (each) comprise 10-100 nucleotides.

The second RNA molecule may comprise at least 50, at least 75 nucleotides, at least 100, at least 105 nucleotides, at least 110 nucleotides, at least 115 nucleotides, at least 120 nucleotides, at least 125 nucleotides, at least 135 nucleotides, at least 145 nucleotides at least 150, at least 155 nucleotides, at least 160 nucleotides, at least 165 nucleotides, at least 170 nucleotides, at least 175 nucleotides, at least 180 nucleotides, at least 185 nucleotides, at least 190 nucleotides, at least 195 nucleotides, at least 200, at least 210, at least 220, at least 230, at least 240, at least 250, at least 260, at least 270, at least 280, at least 290, at least 300, at least 325, at least 350, at least 375, at least 400, at least 450, at least 500, at least 600, at least 700, at least 800, at least 900, at least 1000, at least 1500, at least 2000, at least 3000, at least 4000, at least 5000, at least 6000, at least 7000, at least 8000, or at least 9000 nucleotides. Preferably, the second RNA molecule comprises at least 105 nucleotides.

### 2. Methods for producing an adaptor-ligated DNA product as a template for IVT

The methods may comprise producing an adaptor-ligated DNA product as a template for in vitro transcription (IVT) to produce the second RNA molecule.

The methods may comprise producing an adaptor-ligated DNA product, wherein the method comprises:
(a) contacting an amplified DNA product with an endonuclease and first and second adaptor molecules to form a single contiguous aqueous volume; and
(b) incubating the single contiguous aqueous volume to generate an adaptor-ligated DNA product, wherein the adaptor-ligated DNA product comprises a linear double-stranded region, and wherein the first adaptor molecule is appended to a first end of the linear double-stranded region and the second adaptor molecule is appended to a second end of the linear double-stranded region.

Preferably, the step of contacting the amplified DNA product with the endonuclease, the ligase and first and second adaptor molecules is performed in a single reaction (i.e. a single step).

The first and second adaptor molecules may be identical molecules or they may be different molecules. For example, the first adaptor molecule and/or the second adaptor molecule may comprise a hairpin. The first adaptor molecule and/or the second adaptor molecule may be double-stranded linear nucleic acid molecules comprising one or more nuclease-resistant nucleotides. The first adaptor molecule may comprise a hairpin and the second adaptor molecule may be a double-stranded linear nucleic acid molecule comprising one or more nuclease-resistant nucleotides. Thus, the adaptor-ligated DNA product may be resistant to nuclease (e.g. exonuclease) digestion.

The step of contacting the amplified DNA product with the endonuclease and first and second adaptor molecules is preferably performed in the presence of a ligase. Thus, the invention provides a method for producing an adaptor-ligated DNA product, wherein the method comprises:
(a) contacting the amplified DNA product with an endonuclease, a ligase and first and second adaptor molecules to form a single contiguous aqueous volume; and
(b) incubating the single contiguous aqueous volume to generate the adaptor-ligated DNA product, wherein the adaptor-ligated DNA product comprises a linear double-stranded region, and wherein the first adaptor molecule is appended to a first end of the linear double-stranded region and the second adaptor molecule is appended to a second end of the linear double-stranded region.

The appending (or linking or closing) of the first adaptor molecule and/or the second adaptor molecule may be performed by hybridization or ligation of the adaptor molecules to the ends of the digested DNA product. The digested DNA product may comprise or consist of a linear double-stranded region. Thus, the first adaptor molecule may be hybridized to the first end of the linear double-stranded region. The second adaptor molecule may be hybridized to the second end of the linear double-stranded region. The first adaptor molecule may be ligated to the first end of the linear double-stranded region. The second adaptor molecule may be ligated to the second end of the linear double-stranded region. The appending of the first adaptor molecule and the second adaptor molecule may be performed by both hybridization and ligation of the adaptor molecules to the ends of the linear double-stranded region. Thus, the first adaptor molecule may be hybridized and ligated to the first end of the linear double-stranded region. The second adaptor molecule may be hybridized and ligated to the second end of the linear double-stranded region. The appending may be performed via a linker or spacer molecule which facilitates joining of the adaptor molecule to the first and/or second end of the linear double-stranded region.

The linker or spacer may be at least 10, at least 20, at least 30, at least 40, at least 50, at least 60, at least 70, at least 80, at least 90, at least 100, at least 125, at least 150, at least 175, or at least 200 base pairs long.

The methods may comprise producing an adaptor-ligated DNA product, wherein the method comprises:
(a) amplifying a DNA template molecule as described herein, wherein the DNA template molecule comprises at least one endonuclease target sequence to generate amplified DNA product;
(b) contacting the amplified DNA product with an endonuclease, a ligase and first and second adaptor molecules to form a single contiguous aqueous volume; and
(c) incubating the single contiguous aqueous volume to generate the adaptor-ligated DNA product, wherein the adaptor-ligated DNA product comprises a linear double-stranded region, and wherein the first adaptor molecule is appended to a first end of the linear double-stranded region and the second adaptor molecule is appended to a second end of the linear double-stranded region.

The methods may further comprise, after the step of amplification and before the step of contacting the amplified DNA product with an endonuclease, a ligase and first and second adaptor molecules, a step of heat-deactivation. Thus, the invention provides a method for producing an adaptor-ligated DNA product, the method comprises:
(a) amplifying a DNA template molecule as described herein, wherein the DNA template molecule comprises at least one endonuclease target sequence to generate amplified DNA product;
(b) heat-deactivation of the reaction of step (a);
(c) contacting the amplified DNA product with an endonuclease, a ligase and first and second adaptor molecules to form a single contiguous aqueous volume; and
(d) incubating the single contiguous aqueous volume to generate the adaptor-ligated DNA product, wherein the adaptor-ligated DNA product comprises a linear double-stranded region, and wherein the first adaptor molecule is appended to a first end of the linear double-stranded region and the second adaptor molecule is appended to a second end of the linear double-stranded region.

The step of heat-deactivation may be performed under conditions sufficient to inactive the reagents used during the amplification reaction. The step of heat-deactivation may be performed at a temperature of at least 50°C, at least 55°C, at least 60°C, at least 65°C, at least 70°C, at least 75°C, at least 80°C, at least 85°C, at least 90°C, at least 95°C, or at least 100°C. The step of heat-deactivation may be performed for at least 1 min, at least 3 mins, at least 5 mins, at least 10 mins, at least 15 mins, or at least 20 mins.

In the method described herein, after the step of amplification, the step of contacting the amplified DNA product with an endonuclease, a ligase and first and second adaptor molecules to form a single contiguous aqueous volume may be performed without purifying the product of the amplification reaction. That is to say that the step of contacting the amplified DNA product with an endonuclease, a ligase and first and second adaptor molecules may be performed directly after the step of amplification. The step of contacting the amplified DNA product with an endonuclease, a ligase and first and second adaptor molecules may be performed directly after the step of heat-deactivation.

The method may further comprise, after the step of incubating the single contiguous aqueous volume, a step of purification of the adaptor-ligated DNA product.

The method may further comprise, after the step of incubating the single contiguous aqueous volume, a step of nuclease digestion. The nuclease digestion may be exonuclease digestion, such as exonuclease I and/or exonuclease III digestion. The step of nuclease digestion may take place before or after the step of purification. The step of nuclease digestion may allow for removal of any double-stranded DNA molecules and/or adaptor molecules which have not been used to produce adaptor-ligated DNA product. Thus, the method for producing an adaptor-ligated DNA product may comprise the steps:
(a) contacting the amplified DNA product with an endonuclease, a ligase and first and second adaptor molecules to form a single contiguous aqueous volume;
(b) incubating the single contiguous aqueous volume to generate the adaptor-ligated DNA product, wherein the adaptor-ligated DNA product comprises a linear double-stranded region, and wherein the first adaptor molecule is appended to a first end of the linear double-stranded region and the second adaptor molecule is appended to a second end of the linear double-stranded region; and
(c) incubating the single contiguous aqueous volume with a nuclease (e.g. an exonuclease).

The method for producing an adaptor-ligated DNA product may comprise the steps:
(a) amplifying a DNA template molecule as described herein, wherein the DNA template molecule comprises at least one endonuclease target sequence to generate amplified DNA product;
(b) contacting the amplified DNA product with an endonuclease, a ligase and first and second adaptor molecules to form a single contiguous aqueous volume;
(c) incubating the single contiguous aqueous volume to generate the adaptor-ligated DNA product, wherein the adaptor-ligated DNA product comprises a linear double-stranded region, and wherein the first adaptor molecule is appended to a first end of the linear double-stranded region and the second adaptor molecule is appended to a second end of the linear double-stranded region; and
(d) incubating the single contiguous aqueous volume with a nuclease (e.g. an exonuclease).

The method for producing an adaptor-ligated DNA product may comprise the steps:
(a) amplifying a DNA template molecule as described herein, wherein the DNA template molecule comprises at least one endonuclease target sequence;
(b) contacting the amplified DNA product with an endonuclease, a ligase and first and second adaptor molecules to form a single contiguous aqueous volume;
(c) incubating the single contiguous aqueous volume to generate the adaptor-ligated DNA product, wherein the adaptor-ligated DNA product comprises a linear double-stranded region, and wherein the first adaptor molecule is appended to a first end of the linear double-stranded region and the second adaptor molecule is appended to a second end of the linear double-stranded region;
(d) purification of the adaptor-ligated DNA product; and
(e) incubating the product of step (d) with a nuclease (e.g. an exonuclease).

The method for producing an adaptor-ligated DNA product may comprise the steps:
(a) amplifying a DNA template molecule as described herein, wherein the DNA template molecule comprises at least one endonuclease target sequence;
(b) contacting the amplified DNA product with an endonuclease, a ligase and first and second adaptor molecules to form a single contiguous aqueous volume;
(c) incubating the single contiguous aqueous volume to generate the adaptor-ligated DNA product, wherein the adaptor-ligated DNA product comprises a linear double-stranded region, and wherein the first adaptor molecule is appended to a first end of the linear double-stranded region and the second adaptor molecule is appended to a second end of the linear double-stranded region;
(d) incubating the single contiguous aqueous volume with a nuclease (e.g. an exonuclease); and
(e) purification of the DNA product.

The endonuclease may be a restriction endonuclease (such as a Type II restriction endonuclease), RNA-guided DNA endonuclease, meganuclease or homing endonuclease. The Type II restriction endonuclease may be a Type IIP restriction endonuclease (e.g. EcoRl, Hindlll, BamHI or Notl) or a Type IIS restriction endonuclease (e.g. Bsal, Bbvl, Fokl or Sapl). The RNA-guided DNA endonuclease may be an endonuclease of Class 2 e.g. Class 2 Type V. The RNA-guided DNA endonuclease may be Cas12a. The RNA-guided DNA endonuclease may be Cpf1 or Mad7. Preferably, the RNA-guided DNA endonuclease is Cpf1. The homing endonuclease may be I-Ceul, I-Scel, Pl-Pspl or Pl-Scel.

The endonuclease may be a restriction enzyme endonuclease. The endonuclease may be a Type IIS restriction enzyme. The endonuclease may be any enzyme that recognizes a DNA sequence and cleaves outside of the recognition sequence. For example, the endonuclease may be a Bbsl, Bsal, BsmBI, BspQl, BtgZl, Esp3I,SapI, Aarl, Acc36I, AcIWI, Acul, Ajul, Alol, Alw26I, Alwl, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1I, Bse3DI, BseGI, BseMI, BseMII, BseNI, BseRl, BseXI, Bsgl, BsIFI, BsmAl, BsmFl, Bsml, Bso31I, BspCNI, BspMI, BspPl, BspQl, BspTNI, BsrDl, Bsrl, Bst6I, BstF5I, BstMAI, BstV1I, BstV2I, Bsul, BtgZl, BtsCl, Btsl-v2, BtsMutl, Bvel, Csel, CspCI, Eam1104I, Earl, Ecil, Eco31I, Eco57I, Esp3l, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAIII, PaqCl, PciSI, Pctl, PleI, Ppsl, Psrl, Schl, SfaNl, Taqll, TspDTI and/or TspGWI restriction enzyme.

Type IIS restriction endonucleases cleave the amplified DNA molecule outside of the recognition sequence (i.e. an endonuclease target sequence), which means that the recognition sequence (i.e. endonuclease target sequence) is not included in the adaptor-ligated DNA product.

The step of incubating the single contiguous aqueous volume may be performed under conditions that promote appending (or linking) of the first and second adaptor molecules to the digested DNA product. The appending may be performed by creating a covalent link between the first and/or second adaptor molecule and the first and/or second end of the digested DNA product.

The step of incubating the single contiguous aqueous volume may be performed under conditions that promote digestion of the amplified DNA product to produce the digested DNA product. The digestion of the amplified DNA product to produce the digested DNA product may be performed at a first temperature of 1°C-100°C, 1°C -80°C, 5°C-70°C, 10°C-60°C, 15°C-55°C, 20°C-50°C, 25°C -45°C, 30°C-40°C, 35°C-39°C, 36°C-38°C, or at about 37°C. The digestion may be endonuclease digestion, preferably Type IIS endonuclease digestion.

The step of incubating the single contiguous aqueous volume may be performed under conditions that promote ligation of the digested DNA products to the first and second adaptor molecules. The ligation may be at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% efficient. For example, at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% of the digested DNA products may be incorporated into adaptor-ligated DNA products. Preferably, the ligation is at least 15% efficient.

Ligation efficiency may be established based on DNA quantification values before and after the digestion/ligation reaction. Thus, ligation efficiency may be established based on the equation: (starting amplified DNA amount) / (final linear DNA amount) x 100%.

Ligation efficiency may also be established based on DNA quantification values before and after the digestion/ligation reaction and the subsequent exonuclease treatment to remove remaining DNA constructs and adaptor molecules excess.

For example, the amplified DNA molecule generated by amplification (e.g. rolling circle amplification) may first be quantified so that the amount of the starting material during the digestion/ligation reaction is known. After all the enzymatic reactions, the adaptor-ligated DNA product may be quantified to calculate the ligation efficiency as per the equation above.

DNA quantification methods are known to a person skilled in the art. For example, DNA quantifications may be carried out using the Qubit dsDNA BR assay from ThermoFisher (https://www.thermofisher.com/order/catalog/product/Q32850#/Q32850).

The step of ligation of the digested DNA product to the first and second adaptor molecules may be performed at a second temperature of 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C.

The step of incubating the single contiguous aqueous volume may comprise incubating at a first temperature and then incubating at a second temperature. The first temperature may be 1°C-100°C, 1°C-80°C, 5°C-70°C, 10°C-60°C, 15°C-55°C, 20°C-50°C, 25°C-45°C, 30°C-40°C, 35°C-39°C, 36°C-38°C, or about 37°C. The second temperature may be 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C. Preferably, the first temperature is 35°C-39°C and the second temperature is 14°C-18°C. Using these conditions the endonuclease may be a Type IIS restriction endonuclease (e.g. Bsal) and the ligase may be T4 DNA ligase, T7 DNA ligase, mammalian DNA ligase I, III and IV; Taq DNA ligase, Tth DNA ligase, or E. *coli* DNA ligase.

The step of incubating the single contiguous aqueous volume may be performed isothermally. The step of incubating the single contiguous aqueous volume may comprise incubating at a constant temperature. The constant temperature promotes simultaneous digestion of the amplified DNA product to produce the digested DNA product and ligation of the digested DNA product to the first and second adaptor molecules. For example, the constant temperature may be 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, or 40°C. Preferably, the constant temperature is 30°C. The constant temperature is intended to mean that the temperature does not significantly change during the reaction. The constant temperature is intended to mean that the temperature variation during the step of incubating the single contiguous aqueous volume is less than 10°C, less than 9°C, less than 8°C, less than 7°C, less than 6°C, less than 5°C, less than 4°C, less than 3°C, less than 2°C, or less than 1°C. In a preferred embodiment the temperature during the step of incubating the single contiguous aqueous does not deviate by more than 5°C, preferably by not more than 3°C, even more preferably not more than 1°C. Thus, the constant temperature may be a temperature in a range of 20°C-30°C, 22°C-32°C, 24°C-34°C, 26°C-36°C, 28°C-38°C, 30°C-40°C, 22°C-28°C, 32°C-38°C, 25°C-35°C, 26°C-34°C, 27°C- 33°C, 27.5°C-32.5°C, 28°C-32°C, 28.5°C-31.5°C, 29°C-31°C, or 29.5°C-30.5°C. Preferably, the constant temperature is a temperature in a range of 27.5°C-32.5°C. Alternatively, the constant temperature may be a temperature in a range of 32°C-42°C, 33°C-41°C, 34°C-40°C, 35°C-39°C, 36°C-38°C. Preferably, the constant temperature is a temperature in a range of 34.5°C-39.5°C.

The step of incubating the single contiguous aqueous volume may comprise cycling between the first temperature and the second temperature. The step of incubating the single contiguous aqueous volume may comprise cycling between the first temperature and the second temperature at least 2, at least 3, at least 4, at least 5, at least 6, at least 7, at least 8, at least 9, at least 10, at least 15, at least 20, at least 25, at least 30, at least 35, at least 40, at least 45, at least 50, at least 55, at least 60, at least 65, at least 70, at least 80, at least 90, or at least 100 times, preferably at least 20 times. The step of incubating the single contiguous aqueous volume may comprise cycling between the first temperature and the second temperature less than 40, less than 35, less than 30 times, less than 29, less than 25 times. The step of incubating the single contiguous aqueous volume may comprise cycling between the first temperature and the second temperature 2-100, 5-80, 10-70, 20-60, or 30-60 times. The step of incubating the single contiguous aqueous volume may comprise cycling between the first temperature and the second temperature 2-20, 5-29, 61-100, or 65-80 times.

The DNA template molecule may comprise at least one endonuclease target sequence. Preferably, the DNA template molecule comprises at least two endonuclease target sequences. The endonuclease target sequences may be the same or different. Preferably, the at least one endonuclease target sequence is a restriction endonuclease target sequence. Different restriction endonuclease target sequences would be known to the skilled person. The endonuclease target sequence may be a Type IIS restriction endonuclease target sequence. For example, the restriction endonuclease target sequence may be a Bbsl, Bsal, BsmBI, BspQl, BtgZl, Esp3I,SapI, Aarl, Acc36I, AcIWI, Acul, Ajul, Alol, Alw26I, Alwl, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1I, Bse3DI, BseGI, BseMl, BseMll, BseNI, BseRl, BseXl, Bsgl, BsIFI, BsmAl, BsmFl, Bsml, Bso31I, BspCNI, BspMI, BspPl, BspQl, BspTNI, BsrDl, Bsrl, Bst6I, BstF5I, BstMAI, BstV1I, BstV2I, Bsul, BtgZl, BtsCl, Btsl-v2, BtsMutl, Bvel, Csel, CspCI, Eam1104I, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAIII, PaqCl, PciSI, Pctl, PleI, Ppsl, Psrl, Schl, SfaNI, Taqll, TspDTI and/or TspGWI target sequence. The at least one endonuclease target sequence may be a native endonuclease target sequence (i.e. an endonuclease target sequence present in the template molecule). Alternatively, the at least one endonuclease target sequence may be introduced to the DNA template molecule prior to the production of the adaptor-ligated DNA product.

The endonuclease may be a restriction enzyme endonuclease. The endonuclease may be a Type IIS restriction enzyme. The endonuclease may be any enzyme that recognizes a DNA sequence and cleaves outside of the recognition sequence. For example, the endonuclease may be a Bbsl, Bsal, BsmBI, BspQl, BtgZl, Esp3I,SapI, Aarl, Acc36I, AcIWI, Acul, Ajul, Alol, Alw26I, Alwl, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1I, Bse3DI, BseGI, BseMl, BseMII, BseNI, BseRl, BseXI, Bsgl, BsIFI, BsmAl, BsmFl, Bsml, Bso31I, BspCNI, BspMI, BspPl, BspQl, BspTNI, BsrDl, Bsrl, Bst6I, BstF5I, BstMAI, BstV1I, BstV2I, Bsul, BtgZl, BtsCl, Btsl-v2, BtsMutl, Bvel, Csel, CspCI, Eam1104I, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAIII, PaqCl, PciSI, Pctl, PleI, Ppsl, Psrl, Schl, SfaNl, Taqll, TspDTI and/or TspGWI restriction enzyme.

The ligase may be a DNA ligase, such as a T4 DNA ligase, T7 DNA ligase, mammalian DNA ligase I, III and IV; Taq DNA ligase, Tth DNA ligase, or E. *coli* DNA ligase.

The first adaptor molecule and/or the second adaptor molecule may comprise one or more locked nucleic acids (LNAs).

The first adaptor molecule and/or the second adaptor molecule may comprise one or more protected nucleotides (i.e. nuclease-resistant nucleotides), such as phosphorothioated nucleotides.

The first and second adaptor molecules may comprise one or more phosphorothioated nucleotides, such that, once the adaptor molecules are appended (e.g. ligated) to the digested DNA product, the adaptor-ligated DNA product is resistant to nuclease digestion or has improved or enhanced resistance to nuclease digestion. The adaptor-ligated DNA product may be resistant to 3'-end exonuclease digestion (e.g. by exonuclease III) and/or 5'-end exonuclease digestion (e.g. by exonuclease VIII).

The adaptor molecule may comprise a plurality of phosphorothioated nucleotides. For example, the adaptor molecules may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides in each strand.

The adaptor molecule may be a nucleic acid adaptor molecule. The adaptor molecule may be double-stranded. The adaptor molecule may comprise a portion that is double-stranded.

The first and/or second adaptor molecules may comprise at least 3, at least 4, at least 5, at least 6, at least 7, at least 8, at least 9, at least 10, at least 11, at least 12, at least 13, at least 14, at least 15, or at least 16 base pairs.

The adaptor molecule may comprise a plurality of phosphorothioated nucleotides in each strand. For example, the adaptor molecules may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides in each strand.

The adaptor molecule may comprise a plurality of phosphorothioated nucleotides at internal positions in each strand. For example, the adaptor molecules may comprise at least 1, at least 2, at least 3, at least 4, at least 5, at least 6, at least 7, at least 8, at least 9, at least 10, at least 11, at least 12, at least 13, at least 14, at least 15 or at least 16 phosphorothioated nucleotides at internal positions in each strand. Preferably, the adaptor molecule comprises at least 2 phosphorothioated nucleotides at internal positions in each strand.

The internal positions may not be located between the second and penultimate nucleotide of the adaptor molecule. The internal positions may be any position in the adaptor molecules other than the last nucleotide at the end of each strand.

The adaptor molecule may comprise at least 5%, at least 10%, at least 20%, at least 30%, at least 40%, at least 50%, at least 60%, at least 70%, at least 80%, at least 90%, at least 95%, or at least 100% of protected nucleotides.

The nucleotides resistant to exonuclease digestion (i.e. protected nucleotides) may be phosphorothioated nucleotides of at least one type. For example, the at least one type of phosphorothioated nucleotides is α-S-dATP (i.e. 2'-deoxyadenosine-5'-(a-thio)-triphosphate), α-S-dCTP (i.e. 2'-deoxycytidine-5'-(a-thio)-triphosphate), α-S-dGTP (i.e. 2'-deoxyguanosine-5'-(α-thio)-triphosphate), α-S-dTTP (i.e. 2'-deoxythymidine-5'-(α-thio)-triphosphate), α-S-dUTP (i.e. 2'-deoxyuridine-5'-(α-thio)-triphosphate), and/or uridine 2', 3'-cyclophosphorothioate.

The adaptor molecules may comprise at least two types of phosphorothioated nucleotides. For example, the at least two types of phosphorothioated nucleotides are: α-S-dATP and α-S-dCTP, α-S-dATP and α-S-dGTP, α-S-dATP and α-S-dTTP, α-S-dCTP and α-S-dGTP, α-S-dCTP and α-S-dTTP, or α-S-dGTP and α-S-dTTP.

The adaptor molecules may comprise at least three types of phosphorothioated nucleotides. For example, the at least three types of phosphorothioated nucleotides are:
a. α-S-dATP, α-S-dCTP and α-S-dGTP;
b. α-S-dATP, α-S-dCTP and α-S-dTTP;
c. α-S-dATP, α-S-dGTP and α-S-dTTP; or
d. α-S-dCTP, α-S-dGTP and α-S-dTTP.

The adaptor molecules may comprise at least four types of phosphorothioated nucleotides. For example, the at least four types of protected nucleotides are α-S-dATP, α-S-dCTP, α-S-dGTP and α-S-dTTP.

The phosphorothioated nucleotides may be Sp-isomers, Rp-isomers or a mixture of both Sp- and Rp-isomers.

The nucleotides resistant to exonuclease digestion (i.e. protected nucleotides) may be MOE nucleotides of at least one type, or at least two, three or four types. For example, the MOE nucleotides may be 2'-O-methoxy-ethyl guanosine, 2'-O-methoxy-ethyl cytidine, 2'-O-methoxy-ethyl adenosine, and/or 2'-O-methoxy-ethyl thymidine.

The first end of the digested DNA product may be complementary to a portion of the first adaptor molecule. The second end of the digested DNA product may be complementary to a portion of the second adaptor molecule. The first end and/or the second end of the digested DNA product may be generated by endonuclease digestion as described herein.

As used herein, the term "complementary" refers to the pairing of nucleotide sequences according to Watson/Crick pairing rules. For example, a sequence 5'-GCGGTCCCA-3' has the complementary sequence of 5'-TGGGACCGC-3'. A complement sequence can also be a sequence of RNA complementary to the DNA sequence.

The first adaptor molecule and/or the second adaptor molecule may confer resistance to the nuclease digestion, such as exonuclease digestion (e.g. exonuclease I and/or exonuclease III digestion).

The first adaptor molecule may be a nucleic acid adaptor molecule. The second adaptor molecule may be a nucleic acid adaptor molecule. The first adaptor molecule and/or the second adaptor molecule may comprise a self-complementary element which creates a loop, such as a hairpin loop or a stem loop. Thus, the first adaptor molecule may comprise a hairpin or a stem-loop. The second adaptor molecule may comprise a hairpin or a stem-loop. Both the first and second adaptor molecules may comprise a hairpin or a stem-loop. The adaptor molecules may each comprise a double-stranded portion comprising a sense strand and an antisense strand, wherein the sense strand and the antisense strand are linked together in a hairpin such that the sense strand is hybridized to the antisense strand. The double-stranded portion of an adaptor may comprise a 3' overhang or a 5' overhang of at least 1, at least 2, at least 3, at least 4, or at least 5 nucleotides. Preferably the 3' overhang or the 5' overhang is 4-8 nucleotides. Each end of the linear double-stranded region (or linear portion of the double-stranded DNA molecule) may comprise a 3' or a 5' overhang. A portion of the first adaptor molecule (e.g. the overhang) may be complementary to the first end of the linear double-stranded region. A portion of the second adaptor molecule may be complementary to the second end of the linear double-stranded region.

The adaptor-ligated DNA product may be an open linear DNA product, optionally wherein the linear DNA product comprises a first adaptor molecule at a first end and a second adaptor molecule at a second end. The adaptor-ligated DNA product may be a partially closed linear DNA, optionally wherein the linear double-stranded region is closed at a first end by a first adaptor molecule or closed at a second end by a second adaptor molecule. A partially closed linear DNA product may result from one of the first or second adaptor molecules being a linear double-stranded DNA molecule and other comprising a hairpin or stem-loop. The adaptor-ligated DNA product may be closed linear DNA product, optionally wherein the linear double-stranded region is closed at a first end by a first adaptor molecule and closed at a second end by a second adaptor molecule. A closed linear DNA product may result from both the first and second adaptor molecules comprising a hairpin or stem-loop. Preferably the adaptor-ligated DNA product is a partially closed linear DNA product.

Linear double-stranded adaptors may comprise nuclease-resistant nucleotides as described herein thereby conferring exonuclease resistance on the adaptor-ligated DNA product. Methods for producing closed linear DNA products, linear double-stranded DNA products and partially closed linear DNA products are provided in WO2023/006978A1, which is incorporated herein by reference.

### 3. Protected splint DNA and methods for producing a protected splint DNA

The protected splint DNA may have enhanced resistance to exonuclease digestion. The enhanced resistance to exonuclease digestion may extend the life of the ssDNA cassette in a cell-free system (i.e. the ssDNA cassette may have enhanced resistance to extracellular exonucleases). The ssDNA cassette may be protected from digestion by exonucleases that cleave the 3'-end nucleotides (e.g. exonuclease I), exonucleases that cleave the 5'-end nucleotides (e.g. exonuclease VIII) and exonucleases that cleave both the 3' and 5' end nucleotides (e.g. exonuclease VII).

The protected splint DNA comprising a single-stranded DNA (ssDNA) cassette may comprise a nuclease-resistant nucleotide at the 5' end of the ssDNA cassette or 5' of the ssDNA cassette, and a nuclease-resistant nucleotides at the 3'-end of the ssDNA cassette or 3' of the ssDNA cassette, wherein the cassette comprises at least 100 nucleotides. The protected splint DNA comprising a single-stranded DNA (ssDNA) cassette may comprise at least 3 nuclease-resistant nucleotides at the 5' end of the ssDNA cassette or 5' of the ssDNA cassette, and at least 3 nuclease-resistant nucleotides at the 3'-end of the ssDNA cassette or 3' of the ssDNA cassette, optionally wherein the ssDNA cassette comprises at least 100 nucleotides. The protected splint DNA comprising a single-stranded DNA (ssDNA) cassette may comprise at least 5 nuclease-resistant nucleotides at the 5' end of the ssDNA cassette or 5' of the ssDNA cassette, and at least 5 nuclease-resistant nucleotides at the 3'-end of the ssDNA cassette or 3' of the ssDNA cassette, optionally wherein the ssDNA cassette comprises at least 100 nucleotides.

The protected splint DNA comprising a single-stranded DNA (ssDNA) cassette may comprise x nuclease-resistant nucleotides at the 5' end of the ssDNA cassette or 5' of the ssDNA cassette, and y nuclease-resistant nucleotides at the 3'-end of the ssDNA cassette or 3' of the ssDNA cassette, wherein x is at least 1 and y is at least 1, optionally wherein the ssDNA cassette comprises at least 100 nucleotides.

x and y may be independently selected from at least 1, at least 2, at least 3, at least 4 and at least 5. x and y may be different or the same. Preferably x and y are both at least 3. x and y may both be at least 5. x may be 3 and y may be 1 and *vice versa.* x may be 3 and y may be 5 and *vice versa.*

"Protected splint DNA" as used herein refers to the protected splint DNA comprising a single-stranded DNA (ssDNA) cassette unless specified otherwise.

The protected splint DNA may be resistant to 3'-end exonuclease digestion (e.g. by exonuclease III) and/or 5'-end exonuclease digestion (e.g. by exonuclease VIII). The protected splint DNA may be resistant to 3'-end exonuclease digestion (e.g. by exonuclease III, exonuclease T and/or exonuclease VII) and/or 5'-end exonuclease digestion (e.g. by exonuclease VIII, exonuclease VII, RecJf, RecJ, T7 exonuclease, Lambda exonuclease and/or T5 exonuclease).

The protected splint DNA comprises nuclease-resistant nucleotides (i.e. protected nucleotides), such as phosphorothioated nucleotides.

The protected DNA may be a single-stranded DNA molecule i.e. the protected DNA may not comprise any double-stranded regions.

The protected splint DNA may comprise at least 50, at least 75, at least 100, at least 105, at least 110, at least 115, at least 120, at least 125, at least 130, at least 135, at least 140, at least 145, at least 150, at least 155, at least 160, at least 165, at least 170, at least 175, at least 180, at least 185, at least 190, at least 195, at least 200, at least 250, at least 300, at least 400, at least 500, at least 750, at least 1000 nucleotides, at least 1500, at least 2000, at least 3000, at least 4000, at least 5000, at least 6000, at least 7000, at least 8000, or at least 9000 nucleotides. Preferably, the protected splint DNA comprises at least 135 nucleotides.

The ssDNA cassette may comprise at least 25, at least 50, at least 75, at least 100, at least 105, at least 110, at least 115, at least 120, at least 125 nucleotides, at least 130, at least 135, at least 140, at least 145, at least 150, at least 155, at least 160, at least 165, at least 170, at least 175, at least 180, at least 185, at least 190, at least 195, at least 200, at least 250, at least 300, at least 400, at least 500, at least 750, at least 1000, at least 1500, at least 2000, at least 3000, at least 4000, at least 5000, at least 6000, at least 7000, at least 8000, or at least 9000 nucleotides. Preferably, the ssDNA cassette comprises at least 125 nucleotides.

The ssDNA cassette may be a single cassette. The term "single cassette" as used herein is intended to encompass a product that does not comprise or consist of a plurality of cassettes. That is to say that the protected DNA comprises only a single cassette, which may comprise a single sequence complementary to a pegRNA. The single cassette may not comprise or consist of a plurality of tandem repeat sequences, and/or concatemeric DNA. The term "single cassette" as used herein is intended to encompass a single copy of the DNA sequence of interest, for example, a single copy of the coding sequence. Thus, the "single cassette" may not encompass a cassette that comprises or consist of multiple copies of the same DNA sequence linked in series.

The ssDNA cassette comprises sequences complementary to the sequences of the first RNA molecule, the second RNA molecule and the third RNA molecule.

The protected splint DNA may additionally comprise a plurality of protected nucleotides (e.g. phosphorothioated nucleotides) at internal positions. For example, the protected splint DNA may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, or at least 250 protected nucleotides (e.g. phosphorothioated nucleotides) at internal positions. Preferably, the protected splint DNA comprises at least 2 protected nucleotides (e.g. phosphorothioated nucleotides) at internal positions.

The internal positions may not be located between the second and penultimate nucleotide of the protected splint DNA.

The splint DNA may be a protected splint DNA and the methods may comprise producing the protected splint sDNA comprising a single-stranded DNA (ssDNA) cassette (e.g. a long ssDNA cassette). The method may comprise producing a protected splint DNA comprising a single-stranded DNA (ssDNA) cassette, wherein the protected splint DNA comprises a nuclease-resistant nucleotide at the 5' end of the ssDNA cassette or 5' of the ssDNA cassette, and a nuclease-resistant nucleotide at the 3'-end of the ssDNA cassette or 3' of the ssDNA cassette. The nuclease-resistant nucleotides are preferably exonuclease-resistant nucleotides e.g. phosphorothioated nucleotides. The location of exonuclease-resistant nucleotides at the 5' and 3' ends of and/or outside of the ssDNA cassette protects the ssDNA cassette from exonuclease digestion. For example, it provides protection from digestion by exonucleases that cleave the 3'-end nucleotides (e.g. exonuclease III) and exonucleases that cleave the 5'-end nucleotides (e.g. exonuclease VIII).

The enhanced resistance to exonuclease digestion extends the life of the ssDNA cassette in a cell-free system (i.e. the ssDNA cassette has enhanced resistance to extracellular exonucleases).

The methods may comprise producing a protected splint DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, a nuclease-resistant nucleotide at the 5' end of the cassette or 5' of the cassette, and a nuclease-resistant nucleotide at the 3'-end of the cassette or 3' of the cassette; and
(b) digesting the second strand of the partially protected dsDNA with an exonuclease (or an exonuclease mixture) thereby generating the protected splint DNA.

The invention provides a method for producing a protected DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, at least 3 nuclease-resistant nucleotides at the 5' end of the cassette or 5' of the cassette, and at least 3 nuclease-resistant nucleotides at the 3'-end of the cassette or 3' of the cassette; and
(b) digesting the second strand of the partially protected dsDNA with an exonuclease (or an exonuclease mixture) thereby generating the protected DNA.

The invention provides a method for producing a protected DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, at least 5 nuclease-resistant nucleotides at the 5' end of the cassette or 5' of the cassette, and at least 5 nuclease-resistant nucleotides at the 3'-end of the cassette or 3' of the cassette; and
(b) digesting the second strand of the partially protected dsDNA with an exonuclease (or an exonuclease mixture) thereby generating the protected DNA.

The splint DNA may be a protected DNA comprising a single-stranded DNA (ssDNA) cassette, and wherein the method comprises:
(a) providing a partially protected double-stranded DNA (dsDNA) comprising a first strand and a second strand, wherein the first strand of the partially protected dsDNA comprises a cassette, x nuclease-resistant nucleotides at the 5' end of the cassette or 5' of the cassette, and y nuclease-resistant nucleotides at the 3'-end of the cassette or 3' of the cassette, wherein x is at least 1 and y is at least 1; and
(b) digesting the second strand of the partially protected dsDNA with an exonuclease (or an exonuclease mixture) thereby generating the protected DNA.

The exonuclease (or exonuclease mixture) may comprise at least a 5' to 3' exonuclease and/or a 3' to 5' exonuclease. The exonuclease mixture may comprise at least three exonucleases, wherein at least one of the at least three exonucleases is a 5' to 3' exonuclease and at least one of the at least three exonucleases is a 3' to 5' exonuclease. The exonuclease mixture may comprise at least three exonucleases, wherein at least two of the at least three exonucleases are 5' to 3' exonucleases and at least one of the at least three exonucleases is a 3' to 5' exonuclease. The exonuclease mixture may comprise at least three exonucleases, wherein at least one of the at least three exonucleases is a 5' to 3' exonuclease and at least two of the at least three exonucleases are 3' to 5' exonucleases. The exonuclease mixture may comprise at least four exonucleases, wherein at least one of the at least four exonucleases is a 5' to 3' exonuclease and at least one of the at least four exonucleases is a 3' to 5' exonuclease. The exonuclease mixture may comprise at least four exonucleases, wherein at least two of the at least four exonucleases are 5' to 3' exonucleases and at least one of the at least four exonucleases is a 3' to 5' exonuclease. The exonuclease mixture may comprise at least four exonucleases, wherein at least one of the at least four exonucleases is a 5' to 3' exonuclease and at least two of the at least four exonucleases are 3' to 5' exonucleases. Preferably, the exonuclease mixture comprises at least four exonucleases, wherein at least two of the at least four exonucleases are 5' to 3' exonucleases and at least two of the at least four exonucleases are 3' to 5' exonucleases.

The 5' to 3' exonuclease(s) may be Lambda exonuclease and/or T7 exonuclease. The 3' to 5' exonuclease(s) may be exonuclease I and/or exonuclease III. The 5' to 3' exonuclease(s) may be exonuclease VIII.

The exonuclease mixture may comprise at least two exonucleases selected from a group of exonuclease I, exonuclease III, T7 exonuclease, and Lambda exonuclease. The exonuclease mixture may comprise at least three exonucleases selected from a group of exonuclease I, exonuclease III, T7 exonuclease, and Lambda exonuclease. The exonuclease mixture may comprise exonuclease I, exonuclease III, T7 exonuclease, and Lambda exonuclease.

The exonuclease mixture may comprise one exonuclease selected from Lambda exonuclease and/or T7 exonuclease, and one exonuclease selected from exonuclease I and/or exonuclease III. For example, the exonuclease mixture may comprise Lambda exonuclease and exonuclease I. For example, the exonuclease mixture may comprise Lambda exonuclease and exonuclease III. For example, the exonuclease mixture may comprise T7 exonuclease and exonuclease I. For example, the exonuclease mixture may comprise T7 exonuclease and exonuclease III.

The exonuclease mixture may comprise Lambda exonuclease, T7 exonuclease and exonuclease I. The exonuclease mixture may comprise Lambda exonuclease, T7 exonuclease and exonuclease III. The exonuclease mixture may comprise Lambda exonuclease, exonuclease I and exonuclease III. The exonuclease mixture may comprise T7 exonuclease, exonuclease I and exonuclease III.

The exonuclease mixture may further comprise exonucleases which are able to cleave DNA in both 3' to 5' and 5' to 3' directions. For example, the exonuclease mixture may comprise exonuclease V.

The exonucleases from the exonuclease mixture may be added sequentially or simultaneously. Preferably the exonucleases from the exonuclease mixture are added simultaneously.

x and y may be independently selected from at least 1, at least 2, at least 3, at least 4 and at least 5. x and y may be different or the same. x and y may both be at least 3. x and y may both be at least 5. x may be 3 and y be 1 and *vice versa.* x may be 3 and y be 5 and *vice versa.*

The partially protected dsDNA may be generated by digesting a precursor dsDNA with an endonuclease and ligating first and second adaptor molecules to the digested precursor dsDNA. Preferably, ligation is performed using T7 DNA ligase. The first and second adaptor molecules may comprise exonuclease-resistant nucleotides e.g. phosphorothioated nucleotides.

The methods may comprise producing a protected splint DNA comprising a single-stranded DNA (ssDNA) cassette, wherein the method comprises:
(a) generating a partially protected double-stranded DNA (dsDNA) comprising a first strand and a second strand, wherein the first strand of the partially protected dsDNA comprises a cassette, x nuclease-resistant nucleotides at the 5' end of the cassette or 5' of the cassette, and y nuclease-resistant nucleotides at the 3'-end of the cassette or 3' of the cassette, wherein x is at least 1 and y is at least 1, wherein the step of generating the partially protected dsDNA comprises:
   (i) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
   (ii) digesting the precursor dsDNA with the endonuclease to generate a digested precursor dsDNA;
   (iii) contacting the digested precursor dsDNA with a ligase and first and second adaptor molecules, wherein the first adaptor molecule comprises x nuclease resistant nucleotides and the second adaptor molecule comprises y nuclease-resistant nucleotides and wherein x is at least 1 and y is at least 1, and wherein the ligase is T7 DNA ligase; and
   (iv) ligating the first adaptor molecule to a first end of the digested precursor dsDNA and ligating the second adaptor molecule to a second end of the digested precursor dsDNA thereby generating the partially protected dsDNA; and
(b) digesting the second strand of the partially protected dsDNA with an exonuclease mixture thereby generating the protected splint DNA, wherein the exonuclease mixture comprises at least a 5' to 3' exonuclease and a 3' to 5' exonuclease.

The methods may comprise producing a protected splint DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, x nuclease-resistant nucleotides at the 5' end of the cassette or 5' of the cassette, and y nuclease-resistant nucleotides at the 3'-end of the cassette or 3' of the cassette, wherein x is at least 1 and y is at least 1; and
(b) digesting the second strand of the partially protected dsDNA with an exonuclease mixture thereby generating the protected splint DNA, wherein the exonuclease mixture comprises at least three exonucleases, wherein at least one of the at least three exonucleases is a 5' to 3' exonuclease and at least one of the at least three exonucleases is a 3' to 5' exonuclease.

Importantly, the methods enable the high fidelity and/or high yield production of protected splint DNA comprising long and stable ssDNA cassettes. The methods also enable the production of protected splint DNA of high purity. Methods for producing protected DNA that may be used as protected splint DNA are described in WO2024/017978A1 and EP24383218.5, which are incorporated herein by reference.

The method may be a cell-free method. The method may enable the production of a product comprising the protected DNA that is substantively free of bacterial contaminants (e.g. remaining after cell lysis).

The partially protected double-stranded DNA (dsDNA) may be any partially protected dsDNA defined herein.

Digesting the second strand of the partially protected dsDNA with an exonuclease (or exonuclease mixture) may comprise contacting the partially protected dsDNA with an exonuclease (or exonuclease mixture) to digest the second strand.

Digesting the second strand of the partially protected dsDNA with an exonuclease (or exonuclease mixture) may comprise (a) denaturing the partially protected dsDNA to separate the first and second strands and (b) contacting the second strand with the exonuclease mixture. The partially protected dsDNA may be denatured by means known in the art such as heat and/or alkali conditions.

Heat may include heating the partially protected dsDNA to a temperature of at least 40°C, at least 50°C, at least 60°C, at least 70°C, at least 80°C, or at least 90°C. Preferably, the partially protected dsDNA is heated to at least 95°C. The heating may be carried out for at least 1, at least 3, at least 5, at least 10, at least 15, at least 30 or at least 60 minutes. Preferably, the heating is carried out for at least 3 minutes. Thus, the heating may be at 95°C for at least 3 minutes.

The denatured partially protected dsDNA may be cooled (e.g. to below 20°C, below 10°C or below 5°C) to prevent annealing of the first and second strands. Preferably, the denatured partially protected dsDNA is cooled to around 4°C.

Alkali conditions may be achieved by adding an alkali reagent (e.g. KOH) that creates a pH above 7, 8, 9, 10, 11, 12, 13 or 14. Preferably, an alkali reagent is added that creates a pH above 10. After denaturation, the pH may be lowered by adding an acidic reagent (e.g. HCl). The pH may be lowered by an amount which provides conditions for the exonuclease (or exonuclease mixture) to be effective at digesting the second strand.

If the partially protected dsDNA is denatured, the exonuclease (or exonuclease mixture) may comprise an exonuclease which acts on single-stranded DNA (e.g. *E. coli* exonuclease I, exonuclease VII, exonuclease T, RecJf, RecJ).

The step of exonuclease digestion may allow for the removal of any unprotected strands, strands lacking adaptors at both ends and/or remaining adaptor molecules.

The partially protected dsDNA may be generated by:
(a) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises a nuclease resistant nucleotide and the second adaptor molecule comprises a nuclease-resistant nucleotide; and
(d) ligating the first adaptor molecule to a first end of the digested precursor dsDNA and ligating the second adaptor molecule to a second end of the digested precursor dsDNA thereby generating the partially protected dsDNA.

Preferably, the ligase is T7 DNA ligase.

The partially protected dsDNA may be generated by:
(a) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises at least 3 nuclease resistant nucleotides and the second adaptor molecule comprises at least 3 nuclease-resistant nucleotides; and
(d) ligating the first adaptor molecule to a first end of the digested precursor dsDNA and ligating the second adaptor molecule to a second end of the digested precursor dsDNA thereby generating the partially protected dsDNA.

The partially protected dsDNA may be generated by:
(a) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises at least 5 nuclease resistant nucleotides and the second adaptor molecule comprises at least 5 nuclease-resistant nucleotides; and
(d) ligating the first adaptor molecule to a first end of the digested precursor dsDNA and ligating the second adaptor molecule to a second end of the digested precursor dsDNA thereby generating the partially protected dsDNA.

The partially protected dsDNA may be generated by:
(a) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises x nuclease resistant nucleotides and the second adaptor molecule comprises y nuclease-resistant nucleotides and wherein x is at least 1 and y is at least 1; and
(d) ligating the first adaptor molecule to a first end of the digested precursor dsDNA and ligating the second adaptor molecule to a second end of the digested precursor dsDNA thereby generating the partially protected dsDNA.

The method for producing a protected splint DNA comprising a single-stranded DNA (ssDNA) cassette may comprise:
(a) contacting a precursor dsDNA comprising a first and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first and second adaptor molecules each comprise a nuclease-resistant nucleotide;
(d) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, a nuclease-resistant nucleotide (from the first adaptor molecule) 5' of the cassette, and a nuclease-resistant nucleotide (from the second adaptor molecule) 3' of the cassette;
(e) contacting the partially protected dsDNA with an exonuclease mixture; and
(f) digesting the second strand of the partially protected dsDNA with the exonuclease mixture thereby generating the protected splint DNA.

The method for producing a protected splint DNA comprising a single-stranded DNA (ssDNA) cassette may comprise:
(a) contacting a precursor dsDNA comprising a first and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first and second adaptor molecules each comprise at least 3 nuclease-resistant nucleotides;
(d) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, at least 3 nuclease-resistant nucleotides (from the first adaptor molecule) 5' of the cassette, and at least 3 nuclease-resistant nucleotides (from the second adaptor molecule) 3' of the cassette;
(e) contacting the partially protected dsDNA with an exonuclease mixture; and
(f) digesting the second strand of the partially protected dsDNA with the exonuclease mixture thereby generating the protected splint DNA.

Preferably, the ligase is T7 DNA ligase.

The method for producing a protected splint DNA comprising a single-stranded DNA (ssDNA) cassette may comprise:
(a) contacting a precursor dsDNA comprising a first and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first and second adaptor molecules each comprise at least 5 nuclease-resistant nucleotides;
(d) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, at least 5 nuclease-resistant nucleotides (from the first adaptor molecule) 5' of the cassette, and at least 5 nuclease-resistant nucleotides (from the second adaptor molecule) 3' of the cassette;
(e) contacting the partially protected dsDNA with an exonuclease (or exonuclease mixture); and
(f) digesting the second strand of the partially protected dsDNA with the exonuclease (or exonuclease mixture) thereby generating the protected splint DNA.

The method for producing a protected splint DNA comprising a single-stranded DNA (ssDNA) cassette may comprise:
(a) contacting a precursor dsDNA comprising a first and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises x nuclease-resistant nucleotides and the second adaptor molecule comprises y nuclease-resistant nucleotides, and wherein x is at least 1 and y is at least 1;
(d) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, x nuclease-resistant nucleotides (from the first adaptor molecule) 5' of the cassette, and y nuclease-resistant nucleotides (from the second adaptor molecule) 3' of the cassette;
(e) contacting the partially protected dsDNA with an exonuclease (or exonuclease mixture); and
(f) digesting the second strand of the partially protected dsDNA with the exonuclease (or exonuclease mixture) thereby generating the protected splint DNA.

Steps (a) (i.e. contacting a precursor dsDNA comprising a first and a second strand with an endonuclease) to (d) (i.e. ligating the first and second adaptor molecules to the digested precursor dsDNA) may be performed in a single contiguous aqueous volume.

The precursor dsDNA may comprise one or more endonuclease target sequences. The precursor dsDNA may comprise two endonuclease target sequences. The one or more endonuclease target sequences may be Type IIS endonuclease target sequences. The one or more endonuclease target sequences may be Bbsl, Bsal, BsmBI, BspQl, BtgZl, Esp3I,SapI, Aarl, Acc36I, AcIWI, Acul, Ajul, Alol, Alw26I, Alwl, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1I, Bse3DI, BseGI, BseMl, BseMll, BseNI, BseRl, BseXl, Bsgl, BsIFI, BsmAl, BsmFl, Bsml, Bso31I, BspCNI, BspMI, BspPl, BspQl, BspTNI, BsrDl, Bsrl, Bst6I, BstF5I, BstMAI, BstV1I, BstV2I, Bsul, BtgZl, BtsCl, Btsl-v2, BtsMutl, Bvel, Csel, CspCI, Eam1104I, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAIII, PaqCl, PciSI, Pctl, PleI, Ppsl, Psrl, Schl, SfaNl, Taqll, TspDTI and/or TspGWI target sequences.

The method for producing a protected splint DNA comprising a single-stranded DNA (ssDNA) cassette may comprise:
(a) contacting a precursor dsDNA comprising a first and a second strand with a Type IIS endonuclease, wherein the precursor dsDNA comprises on the first strand a cassette, a Type IIS endonuclease target sequence 5' of the cassette and a Type IIS endonuclease target sequence 3' of the cassette;
(b) digesting the precursor dsDNA with the Type IIS endonuclease to generate a digested precursor dsDNA;
(c) contacting the digested precursor dsDNA with a ligase and first and second adaptor molecules, wherein the first adaptor molecule comprises x nuclease-resistant nucleotides and the second adaptor molecule comprises y nuclease-resistant nucleotides, and wherein x is at least 1 and y is at least 1;
(d) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, x nuclease-resistant nucleotides (from the first adaptor molecule) 5' of the cassette, and y nuclease-resistant nucleotides (from the second adaptor molecule) 3' of the cassette;
(e) contacting the partially protected dsDNA with an exonuclease (or exonuclease mixture); and
(f) digesting the second strand of the partially protected dsDNA with the exonuclease (or exonuclease mixture) thereby generating the protected splint DNA; optionally wherein steps (a) (i.e. contacting a precursor dsDNA comprising a first and a second strand with a Type IIS endonuclease) to (d) (i.e. ligating the first and second adaptor molecules to the digested precursor dsDNA) are performed in a single contiguous aqueous volume.

The precursor dsDNA may be a product of amplification. Preferably, the amplification is rolling circle amplification.

The method may further comprise, before step (a) (i.e. the step of contacting a precursor dsDNA with the endonuclease), a step of amplifying a DNA template to produce the precursor DNA, wherein the DNA template comprises the cassette and the endonuclease target sequences, optionally wherein the DNA template is amplified by rolling circle amplification.

Thus, the method for producing a protected splint DNA comprising a single-stranded DNA (ssDNA) cassette may comprise:
(a) amplifying a DNA template to generate a precursor dsDNA, wherein the DNA template comprises a cassette and an endonuclease target sequence 5' of the cassette and an endonuclease target sequence 3' of the cassette, optionally wherein the DNA template molecule is amplified by rolling circle amplification;
(b) contacting the precursor dsDNA comprising a first and a second strand with an endonuclease, wherein the precursor dsDNA comprises on the first strand the cassette, the endonuclease target sequence 5' of the cassette and the endonuclease target sequence 3' of the cassette;
(c) digesting the precursor dsDNA with the endonuclease to generate a digested precursor dsDNA;
(d) contacting the digested precursor dsDNA with a ligase and first and second adaptor molecules, wherein the first and second adaptor molecules each comprise a nuclease-resistant nucleotide;
(e) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, a nuclease-resistant nucleotide (from the first adaptor molecule) 5' of the cassette, and a nuclease-resistant nucleotide (from the second adaptor molecule) 3' of the cassette;
(f) contacting the partially protected dsDNA with an exonuclease (or exonuclease mixture); and
(g) digesting the second strand of the partially protected dsDNA with the exonuclease (or exonuclease mixture) thereby generating the protected splint DNA.

The method for producing a protected splint DNA comprising a single-stranded DNA (ssDNA) cassette may comprise:
(a) amplifying a DNA template to generate a precursor dsDNA, wherein the DNA template comprises a cassette and an endonuclease target sequence 5' of the cassette and an endonuclease target sequence 3' of the cassette, optionally wherein the DNA template molecule is amplified by rolling circle amplification;
(b) contacting the precursor dsDNA comprising a first and a second strand with an endonuclease, wherein the precursor dsDNA comprises on the first strand the cassette, the endonuclease target sequence 5' of the cassette and the endonuclease target sequence 3' of the cassette;
(c) digesting the precursor dsDNA with the endonuclease to generate a digested precursor dsDNA;
(d) contacting the digested precursor dsDNA with a ligase and first and second adaptor molecules, wherein the first and second adaptor molecules each comprise at least 3 nuclease-resistant nucleotides;
(e) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, at least 3 nuclease-resistant nucleotides (from the first adaptor molecule) 5' of the cassette, and at least 3 nuclease-resistant nucleotides (from the second adaptor molecule) 3' of the cassette;
(f) contacting the partially protected dsDNA with an exonuclease (or exonuclease mixture); and
(g) digesting the second strand of the partially protected dsDNA with the exonuclease (or exonuclease mixture) thereby generating the protected splint DNA.

The method for producing a protected splint DNA comprising a single-stranded DNA (ssDNA) cassette may comprise:
(a) amplifying a DNA template to generate a precursor dsDNA, wherein the DNA template comprises a cassette and an endonuclease target sequence 5' of the cassette and an endonuclease target sequence 3' of the cassette, optionally wherein the DNA template molecule is amplified by rolling circle amplification;
(b) contacting the precursor dsDNA comprising a first and a second strand with an endonuclease, wherein the precursor dsDNA comprises on the first strand the cassette, the endonuclease target sequence 5' of the cassette and the endonuclease target sequence 3' of the cassette;
(c) digesting the precursor dsDNA with the endonuclease to generate a digested precursor dsDNA;
(d) contacting the digested precursor dsDNA with a ligase and first and second adaptor molecules, wherein the first and second adaptor molecules each comprise at least 5 nuclease-resistant nucleotides;
(e) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, at least 5 nuclease-resistant nucleotides (from the first adaptor molecule) 5' of the cassette, and at least 5 nuclease-resistant nucleotides (from the second adaptor molecule) 3' of the cassette;
(f) contacting the partially protected dsDNA with an exonuclease (or exonuclease mixture); and
(g) digesting the second strand of the partially protected dsDNA with the exonuclease (or exonuclease mixture) thereby generating the protected splint DNA.

The method for producing a protected splint DNA comprising a single-stranded DNA (ssDNA) cassette may comprise:
(a) amplifying a DNA template to generate a precursor dsDNA, wherein the DNA template comprises a cassette and an endonuclease target sequence 5' of the cassette and an endonuclease target sequence 3' of the cassette, optionally wherein the DNA template molecule is amplified by rolling circle amplification;
(b) contacting the precursor dsDNA comprising a first and a second strand with an endonuclease, wherein the precursor dsDNA comprises on the first strand the cassette, the endonuclease target sequence 5' of the cassette and the endonuclease target sequence 3' of the cassette;
(c) digesting the precursor dsDNA with the endonuclease to generate a digested precursor dsDNA;
(d) contacting the digested precursor dsDNA with a ligase and first and second adaptor molecules, wherein the first adaptor molecule comprises x nuclease-resistant and wherein the second adaptor molecule comprises y nuclease-resistant nucleotides, and wherein x is at least 1 and y is at least 1;
(e) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, x nuclease-resistant nucleotides (from the first adaptor molecule) 5' of the cassette, and y nuclease-resistant nucleotides (from the second adaptor molecule) 3' of the cassette;
(f) contacting the partially protected dsDNA with an exonuclease (or exonuclease mixture); and
(g) digesting the second strand of the partially protected dsDNA with the exonuclease (or exonuclease mixture) thereby generating the protected splint DNA.

x and y may be independently selected from at least 1, at least 2, at least 3, at least 4 and at least 5. x and y may be different or the same. Preferably x and y are both at least 3. x and y may both be at least 5. x may be 3 and y be 1 and *vice versa.* x may be 3 and y be 5 and *vice versa.*

Steps (b) (i.e. contacting the precursor dsDNA comprising a first and a second strand with an endonuclease) to (e) (i.e. ligating the first and second adaptor molecules to the digested precursor dsDNA) may be performed in a single contiguous aqueous volume.

The step of amplifying a DNA template may be an *in vitro* or *in vivo* amplification. Preferably, the amplification is an *in vitro* amplification. For example, the amplification may be performed by rolling circle amplification (RCA), MALBAC method, traditional polymerase chain reaction (PCR), nucleic acid sequence-based amplification (NASBA), loop-mediated isothermal amplification (LAMP), helicase-dependent amplification (HDA), multiple displacement amplification (MDA) and recombinase polymerase amplification (RPA). Preferably, the amplification is rolling circle amplification.

Rolling circle amplification may be performed without any primers, or in the presence of a primer or multiple primers. For example, the primer may be a synthetic primer. The primers may be random primers. Rolling circle amplification may be performed in the presence of a primase. The primase may be TthPrimPol. Preferably, if the rolling circle amplification is performed without any primers, it is performed in the presence of a primase, such as TthPrimPol. Similarly, if a primer is used during amplification reaction, a primase is not used. The double-stranded DNA product may be generated by the rolling circle amplification in vitro under isothermal conditions using a suitable nucleic acid polymerase, such as Phi29 DNA polymerase.

The method may further comprise a step of purification of the digested precursor dsDNA after digesting the precursor dsDNA.

The method may further comprise a step of purification of the partially protected dsDNA after ligating the first and second adaptor molecules to the digested precursor dsDNA.

The method may further comprise a step of purification of the protected DNA after digesting the second strand of the partially protected dsDNA with an exonuclease (or exonuclease mixture).

The method for producing a protected splint DNA comprising a single-stranded DNA (ssDNA) cassette may comprise:
(a) amplifying a DNA template to generate a precursor dsDNA, wherein the DNA template comprises a cassette and a Type IIS endonuclease target sequence 5' of the cassette and a Type IIS endonuclease target sequence 3' of the cassette, wherein the DNA template molecule is amplified by rolling circle amplification;
(b) contacting the precursor dsDNA comprising a first and a second strand with a Type IIS endonuclease, wherein the precursor dsDNA comprises on the first strand the cassette, the Type IIS endonuclease target sequence 5' of the cassette and the Type IIS endonuclease target sequence 3' of the cassette;
(c) digesting the precursor dsDNA with the Type IIS endonuclease to generate a digested precursor dsDNA;
(d) contacting the digested precursor dsDNA with a ligase and first and second adaptor molecules, wherein the first adaptor molecule comprises x nuclease-resistant and wherein the second adaptor molecule comprises y nuclease-resistant nucleotides, and wherein x is at least 1 and y is at least 1;
(e) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, x nuclease-resistant nucleotides (from the first adaptor molecule) 5' of the cassette, and y nuclease-resistant nucleotides (from the second adaptor molecule) 3' of the cassette;
(f) contacting the partially protected dsDNA with an exonuclease (or exonuclease mixture); and
(g) digesting the second strand of the partially protected dsDNA with the exonuclease (or exonuclease mixture) thereby generating the protected splint DNA; optionally wherein steps (b) (i.e. contacting the precursor dsDNA comprising a first and a second strand with an endonuclease) to (e) (i.e. ligating the first and second adaptor molecules to the digested precursor dsDNA) are performed in a single contiguous aqueous volume.

The partially protected dsDNA may be generated by:
(a) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises a nuclease resistant nucleotide and the second adaptor molecule comprises a nuclease-resistant nucleotide and the first and/or second adaptor molecule comprises an excisable nucleotide; and
(d) ligating the first adaptor molecule to a first end of the digested precursor dsDNA and ligating the second adaptor molecule to a second end of the digested precursor dsDNA thereby generating the partially protected dsDNA.

The partially protected dsDNA may be generated by:
(a) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises at least 3 nuclease resistant nucleotides and the second adaptor molecule comprises at least 3 nuclease-resistant nucleotides and the first and/or second adaptor molecule comprises an excisable nucleotide or an abasic site; and
(d) ligating the first adaptor molecule to a first end of the digested precursor dsDNA and ligating the second adaptor molecule to a second end of the digested precursor dsDNA thereby generating the partially protected dsDNA.

The partially protected dsDNA may be generated by:
(a) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises at least 5 nuclease resistant nucleotides and the second adaptor molecule comprises at least 5 nuclease-resistant nucleotides and the first and/or second adaptor molecule comprises an excisable nucleotide or an abasic site; and
(d) ligating the first adaptor molecule to a first end of the digested precursor dsDNA and ligating the second adaptor molecule to a second end of the digested precursor dsDNA thereby generating the partially protected dsDNA.

The partially protected dsDNA may be generated by:
(a) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises x nuclease resistant nucleotides and the second adaptor molecule comprises y nuclease-resistant nucleotides and wherein x is at least 1 and y is at least 1 and the first and/or second adaptor molecule comprises n excisable nucleotides wherein n is at least 1; and
(d) ligating the first adaptor molecule to a first end of the digested precursor dsDNA and ligating the second adaptor molecule to a second end of the digested precursor dsDNA thereby generating the partially protected dsDNA.

The methods may comprise producing a protected splint DNA comprising a single-stranded DNA (ssDNA) cassette, wherein the method comprises:
(a) contacting a precursor dsDNA comprising a first and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first and second adaptor molecules each comprise a nuclease-resistant nucleotide and the first and/or second adaptor molecule comprises an excisable nucleotide;
(d) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, a nuclease-resistant nucleotide (from the first adaptor molecule) 5' of the cassette, and a nuclease-resistant nucleotide (from the second adaptor molecule) 3' of the cassette and the second strand comprises an excisable nucleotide;
(e) contacting the partially protected dsDNA with a DNA glycosylase and an exonuclease (or exonuclease mixture); and
(f) incubating the partially protected dsDNA with the DNA glycosylase and the exonuclease (or exonuclease mixture) thereby generating the protected splint DNA.

The method for producing a protected splint DNA comprising a single-stranded DNA (ssDNA) cassette may comprise:
(a) contacting a precursor dsDNA comprising a first and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first and second adaptor molecules each comprise at least 3 nuclease-resistant nucleotides and the first and/or second adaptor molecule comprises an excisable nucleotide;
(d) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, at least 3 nuclease-resistant nucleotides (from the first adaptor molecule) 5' of the cassette, and at least 3 nuclease-resistant nucleotides (from the second adaptor molecule) 3' of the cassette and wherein the second strand comprises an excisable nucleotide (from the first and/or second adaptor molecule);
(e) contacting the partially protected dsDNA with a DNA glycosylase and an exonuclease (or exonuclease mixture); and
(f) incubating the partially protected dsDNA with the DNA glycosylase and the exonuclease (or exonuclease mixture) thereby generating the protected splint DNA.

The method for producing a protected splint DNA comprising a single-stranded DNA (ssDNA) cassette may comprise:
(a) contacting a precursor dsDNA comprising a first and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first and second adaptor molecules each comprise at least 5 nuclease-resistant nucleotides and the first and/or second adaptor molecule comprises an excisable nucleotide;
(d) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, at least 5 nuclease-resistant nucleotides (from the first adaptor molecule) 5' of the cassette, and at least 5 nuclease-resistant nucleotides (from the second adaptor molecule) 3' of the cassette and the second strand comprises at least 2 excisable nucleotides (from the first and/or second adaptor molecule);
(e) contacting the partially protected dsDNA with a DNA glycosylase and an exonuclease (or exonuclease mixture); and
(f) incubating the partially protected dsDNA with the DNA glycosylase and the exonuclease (or exonuclease mixture) thereby generating the protected splint DNA.

The methods may comprise producing a protected splint DNA comprising a single-stranded DNA (ssDNA) cassette, wherein the method comprises:
(a) contacting a precursor dsDNA comprising a first and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises x nuclease-resistant nucleotides and n excisable nucleotides and the second adaptor molecule comprises y nuclease-resistant nucleotides and m excisable nucleotides, and wherein x is at least 1 and y is at least 1, wherein n is 0 or at least 1 and m is 0 or at least 1 and n+m is at least 1;
(d) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, x nuclease-resistant nucleotides (from the first adaptor molecule) 5' of the cassette, and y nuclease-resistant nucleotides (from the second adaptor molecule) 3' of the cassette and the second strand comprises n+m excisable nucleotides;
(e) contacting the partially protected dsDNA with a DNA glycosylase and an exonuclease (or exonuclease mixture); and
(f) incubating the partially protected dsDNA with the DNA glycosylase and the exonuclease (or exonuclease mixture) thereby generating the protected splint DNA.

The partially protected dsDNA may be generated by:
(a) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises x nuclease resistant nucleotides and the second adaptor molecule comprises y nuclease-resistant nucleotides and wherein x is at least 1 and y is at least 1 and the first and/or second adaptor molecule comprises n abasic sites wherein n is at least 1; and
(d) ligating the first adaptor molecule to a first end of the digested precursor dsDNA and ligating the second adaptor molecule to a second end of the digested precursor dsDNA thereby generating the partially protected dsDNA.

The methods may comprise producing a protected splint DNA comprising a single-stranded DNA (ssDNA) cassette, wherein the method comprises:
(a) contacting a precursor dsDNA comprising a first and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises x nuclease-resistant nucleotides and n abasic sites and the second adaptor molecule comprises y nuclease-resistant nucleotides and m abasic sites, and wherein x is at least 1 and y is at least 1, wherein n is 0 or at least 1 and m is 0 or at least 1 and n+m is at least 1;
(d) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, x nuclease-resistant nucleotides (from the first adaptor molecule) 5' of the cassette, and y nuclease-resistant nucleotides (from the second adaptor molecule) 3' of the cassette and the second strand comprises n+m abasic sites (from the first and/or second adaptor molecules);
(e) contacting the partially protected dsDNA with an exonuclease (or exonuclease mixture) and optionally with an AP endonuclease; and
(f) incubating the partially protected dsDNA with the exonuclease (or exonuclease mixture) and the optional AP endonuclease thereby generating the protected splint DNA.

The endonuclease may be a restriction enzyme endonuclease. The endonuclease may be a Type IIS restriction enzyme. The endonuclease may be any enzyme that recognizes a DNA sequence and cleaves outside of the recognition sequence. For example, the endonuclease may be a Bbsl, Bsal, BsmBI, BspQl, BtgZl, Esp3I,SapI, Aarl, Acc36I, AcIWI, Acul, Ajul, Alol, Alw26I, Alwl, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1I, Bse3DI, BseGI, BseMl, BseMll, BseNI, BseRl, BseXl, Bsgl, BsIFI, BsmAl, BsmFl, Bsml, Bso31I, BspCNI, BspMI, BspPl, BspQl, BspTNI, BsrDl, Bsrl, Bst6I, BstF5I, BstMAI, BstV1I, BstV2I, Bsul, BtgZl, BtsCl, Btsl-v2, BtsMutl, Bvel, Csel, CspCI, Eam1104I, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAIII, PaqCl, PciSI, Pctl, PleI, Ppsl, Psrl, Schl, SfaNl, Taqll, TspDTI and/or TspGWI restriction enzyme.

Type IIS restriction endonucleases cleave dsDNA outside of the recognition sequence (i.e. an endonuclease target sequence), which means that the recognition sequence (i.e. endonuclease target sequence) is not included in the cleaved product.

The ligase may be a DNA ligase, such as a T4 DNA ligase, T7 DNA ligase, mammalian DNA ligase I, III and IV; Taq DNA ligase, Tth DNA ligase, or E. *coli* DNA ligase. The ligase is preferably T7 DNA ligase.

The DNA glycosylase may be OGG1, MPG, SMUG1, UNG1, MBD4, TDG, MYH1, NTHL1, NEIL1, NEIL2 or NEIL3.

The exonuclease may be an exonuclease which acts on single-stranded DNA (e.g. E. *coli* exonuclease I, exonuclease VII, exonuclease T, RecJf, RecJ). This may be the case when the partially protected dsDNA is denatured.

Alternatively or in combination, the exonuclease may be an exonuclease which acts on double-stranded DNA (e.g. *E. coli* exonuclease III, T7 exonuclease, exonuclease V, exonuclease VIII, T5 exonuclease, lambda exonuclease). This may be the case when the partially protected dsDNA is not denatured.

The protected DNA may be resistant to 3'-end exonuclease digestion (e.g. by exonuclease III) and/or 5'-end exonuclease digestion (e.g. by exonuclease VIII). The protected DNA may be resistant to 3'-end exonuclease digestion (e.g. by exonuclease III, exonuclease I, exonuclease T, exonuclease V and/or exonuclease VII) and/or 5'-end exonuclease digestion (e.g. by exonuclease VIII, RecJf, RecJ, T7 exonuclease, Lambda exonuclease, T5 exonuclease, exonuclease V and/or exonuclease VII).

The step of digesting the precursor dsDNA with the endonuclease to generate a digested precursor dsDNA may be performed by incubating the combined components under conditions that promote digestion of the precursor dsDNA to produce the digested precursor dsDNA. The digestion of the precursor dsDNA to produce the digested precursor dsDNA may be performed at a first temperature of 1°C-100°C, 1°C -80°C, 5°C-70°C, 10°C-60°C, 15°C-55°C, 20°C-50°C, 25°C -45°C, 30°C-40°C, 35°C-39°C, 36°C-38°C, or at about 37°C. The digestion may be endonuclease digestion, preferably Type IIS endonuclease digestion.

The step of ligating the first and second adaptor molecules to the digested precursor dsDNA may be performed by incubating the combined components under conditions that promote ligation of the first and second adaptor molecules to the digested precursor dsDNA to produce the partially protected dsDNA. The ligating may be performed by creating a covalent link between the first and/or second adaptor molecule and the first and/or second end of the digested precursor dsDNA. Preferably, the step of ligation is performed in the presence of T7 DNA ligase.

The ligation may be at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% efficient. Preferably, the ligation is at least 50%, or at least 70% efficient. For example, at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% of the digested precursor dsDNA may be incorporated into partially protected dsDNA. Preferably, at least 25 % or at least 30% of the digested precursor dsDNA is incorporated into partially protected dsDNA

Ligation efficiency may be established based on DNA quantification values before and after the digestion/ligation reaction. Thus, ligation efficiency may be established based on the equation: (starting amplified DNA amount) / (final linear DNA amount) x 100%.

Ligation efficiency may also be established based on DNA quantification values before and after the digestion/ligation reaction and the subsequent exonuclease treatment to remove remaining open DNA constructs and adaptor molecules excess.

For example, the precursor dsDNA generated by the amplification (e.g. rolling circle amplification) is first quantified so that the amount of the precursor dsDNA used as the starting material during the digestion/ligation reaction is known. After all the enzymatic reactions, the partially protected dsDNA is quantified to calculate the ligation efficiency as per the equation above.

DNA quantification methods are known to a person skilled in the art. For example, DNA quantifications may be carried out using the Qubit dsDNA BR assay from ThermoFisher (https://www.thermofisher.com/order/catalog/product/Q32850#/Q32850).

The step of ligating the digested precursor dsDNA to the first and second adaptor molecules may be performed at a second temperature of 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C.

Using the above conditions the endonuclease may be a Type IIS restriction endonuclease (e.g. Bsal) and the ligase may be T4 DNA ligase, T7 DNA ligase, mammalian DNA ligase I, III and IV; Taq DNA ligase, Tth DNA ligase, or *E*. *coli* DNA ligase. Preferably, the ligase is T7 DNA ligase.

The step of digesting the second strand of the partially protected dsDNA with an exonuclease (or exonuclease mixture) may be performed at a temperature of 5-90°C, 10-80°C, 15-70°C, 20-60°C, 25-50°C, 30-45°C or 35-40°C. Preferably, the step of digesting the second strand of the partially protected dsDNA with an exonuclease (or exonuclease mixture) is performed at a temperature of 30-45°C. The step of digesting the second strand of the partially protected dsDNA with an exonuclease (or exonuclease mixture) may be performed for at least 10, at least 20, at least 30, at least 40, at least 50, at least 60 or at least 120 minutes. Preferably, the step of digesting the second strand of the partially protected dsDNA with an exonuclease (or exonuclease mixture) may be performed for at least 60 minutes. For example, the step of digesting the second strand of the partially protected dsDNA with an exonuclease (or exonuclease mixture) may be performed at around 37 °C for at least 120 minutes.

The step of digesting the second strand of the partially protected dsDNA with an exonuclease (or exonuclease mixture) may be followed by inactivating the exonuclease (or exonucleases in the exonuclease mixture), for example by heat-inactivation. Thus, the step of inactivating the exonuclease (or exonucleases in the exonuclease mixture) may be performed at a temperature of 60-90°C, 65-85°C or 70-80°C. Preferably, the step of inactivating the exonuclease (or exonucleases in the exonuclease mixture) is performed at a temperature of 70-80°C. The step of inactivating the exonuclease (or exonucleases in the exonuclease mixture) may be performed at a temperature of at least 60°C, at least 65°C, at least 70°C or at least 80°C. Preferably, the step of inactivating the exonuclease (or exonucleases in the exonuclease mixture) is performed at a temperature of at least 70°C. The step of inactivating the exonuclease (or exonucleases in the exonuclease mixture) may be performed for at least 1, at least 5, at least 10, at least 20 or at least 30 minutes. Preferably, the step of inactivating the exonuclease (or exonucleases in the exonuclease mixture) is performed for at least 5 minutes.

The step of digesting the second strand of the partially protected dsDNA with an exonuclease (or exonuclease mixture) may be performed at a temperature of 30-45°C for at least 60 minutes followed by performing the step of inactivating the exonuclease (or exonucleases in the exonuclease mixture) at a temperature of at least 70°C for at least 5 minutes.

The cassette may comprise at least 25, at least 50, at least 100, at least 150, at least 200, at least 210, at least 220, at least 230, at least 240, at least 250, at least 260, at least 270, at least 280, at least 290, at least 300, at least 325, at least 350, at least 375, at least 400, at least 450, at least 500, at least 600, at least 700, at least 800, at least 900, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 nucleotides. Preferably, the cassette comprises at least 100 nucleotides.

The first and second adaptor molecules may be nucleic acids, optionally DNA. The first and second adaptor molecules may comprise a first strand hybridised to a second strand (e.g. dsDNA).

The first and second adaptor molecules may have different sequences.

The first and second adaptor molecules may comprise dsDNA comprising a first strand and a second strand. The first strands of the first and second adaptor molecules may be ligated by the ligase to the first strand of the digested precursor dsDNA molecule and the second strands of the first and second adaptor molecules may be ligated by the ligase to the second strand of the digested precursor dsDNA molecule.

The first adaptor molecule may be compatible with the first end of the digested precursor dsDNA and incompatible with the second end of the digested precursor dsDNA. The second adaptor molecule may be compatible with the second end of the digested precursor dsDNA and incompatible with the first end of the digested precursor dsDNA.

The first adaptor molecule may be compatible with the 5' end of the first strand of the digested precursor dsDNA and incompatible with the 3' end of the first strand of the digested precursor dsDNA. The second adaptor molecule may be compatible with the 3' end of the first strand of the digested precursor dsDNA and incompatible with the 5' end of the first strand of the digested precursor dsDNA.

The first strand of the first adaptor molecule may be compatible with the 5' end of the first strand of the digested precursor dsDNA and incompatible with the 3' end of the first strand and the 3' and 5' ends of the second strand of the digested precursor dsDNA. The second strand of the first adaptor molecule may be compatible with the 3' end of the second strand of the digested precursor dsDNA and incompatible with the 5' end of the second strand and the 3' and 5' ends of the first strand of the digested precursor dsDNA.

The first strand of the second adaptor molecule may be compatible with the 3' end of the first strand of the digested precursor dsDNA and incompatible with the 5' end of the first strand and the 3' and 5' ends of the second strand of the digested precursor dsDNA. The second strand of the second adaptor molecule may be compatible with the 5' end of the second strand of the digested precursor dsDNA and incompatible with the 3' end of the second strand and the 3' and 5' ends of the first strand of the digested precursor dsDNA.

The second strand of the first and second adaptor molecules may not be resistant to exonuclease digestion. The second strand of the first and second adaptor molecules may be susceptible to exonuclease digestion. The second strand of the first and second adaptor molecules may not comprise any nuclease-resistant nucleotides. The second strand may comprise n excisable nucleotides and the second strand may also comprise protected nucleotides. The protected nucleotides may be at the 3' end of or 3' of the cassette. The second strand may comprise 1, 2, 3, 4, 5, 6, 7 or 8 protected nucleotides at the 3' end or 3' of the cassette, and there may not be protected nucleotides at the 5' end of or 5' of the cassette. The protected nucleotides may be at the 5' end of or 5' of the cassette. The second strand may comprise 1, 2, 3, 4, 5, 6, 7 or 8 protected nucleotides at the 5' end and there may not be protected nucleotides at the 3' end or 3' of the cassette.

The first strand of the first adaptor molecule may comprise the x nucleotide-resistant nucleotides and the first strand of the second adaptor molecule may comprise the y nucleotide-resistant nucleotides. The nucleotide-resistant nucleotide(s) in the first strand of the first adaptor molecule may be located at the 5' or 3' end, preferably the 5' end; and/or the nucleotide-resistant nucleotide(s) in the first strand of the second adaptor molecule may be located at the 5' or 3' end, preferably the 3' end. The nuclease-resistant nucleotide may be a phosphorothioated nucleotide.

The second strand of the first adaptor molecule may comprise the x' nucleotide-resistant nucleotides and the second strand of the second adaptor molecule may comprise the y' nucleotide-resistant nucleotides. The nucleotide-resistant nucleotide(s) in the second strand of the first adaptor molecule may be located at the 5' or 3' end, preferably the 3' end; and/or the nucleotide-resistant nucleotide(s) in the second strand of the second adaptor molecule may be located at the 5' or 3' end, preferably the 5' end. The nuclease-resistant nucleotide may be a phosphorothioated nucleotide.

The first adaptor molecule and/or the second adaptor molecule may be a synthetic adaptor molecule.

The first adaptor molecule and/or the second adaptor molecule may comprise a self-complementary element which creates a loop, such as a hairpin loop or a stem loop. Thus, the first adaptor molecule may comprise a hairpin or a stem-loop. The second adaptor molecule may comprise a hairpin or a stem-loop. Both the first and second adaptor molecules may comprise a hairpin or a stem-loop. The adaptor molecules may each comprise a double-stranded portion comprising a first strand and a second strand, wherein the first strand and the second strand are linked together in a hairpin such that the first strand is hybridized to the second strand. The double-stranded portion of an adaptor may comprise a 3' overhang or a 5' overhang of at least 1, at least 2, at least 3, at least 4, or at least 5 nucleotides. Preferably the 3' overhang or the 5' overhang is 4-8 nucleotides. Each end of the digested precursor dsDNA may comprise a 3' or a 5' overhang. A portion of the first adaptor molecule (e.g. the overhang) may be complementary to the first end of the digested precursor dsDNA. A portion of the second adaptor molecule may be complementary to the second end of the digested precursor dsDNA.

If the first and/or second adaptor molecules comprise a hairpin, following ligation of the first and second adaptor molecules to the digested precursor dsDNA there may be a hairpin 3' and/or 5' of the cassette. The hairpin may link the first and second strands of the digested precursor dsDNA to form a closed loop at one or both ends. Thus, the methods described herein may further comprise nicking a closed loop at one or both ends in order to generate a partially protected dsDNA with a 3' and/or 5' end nucleotide on the second strand.

The first adaptor molecule and/or the second adaptor molecule may comprise an overhang. The first and/or second ends of the digested precursor dsDNA may comprise a 3' or a 5' overhang. A portion of the first adaptor molecule (e.g. the overhang) may be complementary to the first end of the digested precursor dsDNA. A portion of the second adaptor molecule (e.g. the overhang) may be complementary to the second end of the digested precursor dsDNA.

The first adaptor molecule may comprise an overhang which is complementary to the first end of the digested precursor dsDNA. The second adaptor molecule may comprise an overhang which is complementary to the second end of the digested precursor dsDNA.

The first adaptor molecule may comprise an overhang which is non-complementary to the second end of the digested precursor dsDNA. The second adaptor molecule may comprise an overhang which is non-complementary to the first end of the digested precursor dsDNA.

The first adaptor molecule may comprise an overhang complementary to an overhang at the first end of the digested precursor dsDNA and the second adaptor molecule may comprise an overhang complementary to an overhang at the second end of the digested precursor dsDNA. Optionally, the overhang of the first adaptor molecule may not be complementary to the overhang at the second end of the digested precursor dsDNA and the overhang of the second adaptor molecule may not be complementary to the overhang at the first end of the digested precursor dsDNA.

The first adaptor molecule may comprise an overhang that is complementary to and anneals to a 5' overhang of the digested precursor dsDNA and the second adaptor molecule may comprise an overhang that is complementary to and anneals to a 3' overhang of the digested precursor dsDNA.

The first adaptor molecule may comprise an overhang that is complementary to and anneals to a 3' overhang of the digested precursor dsDNA and the second adaptor molecule may comprise an overhang that is complementary to and anneals to a 5' overhang of the digested precursor dsDNA.

The first adaptor molecule and/or the second adaptor molecule may not be a plasmid or a vector DNA.

The first adaptor molecule and/or the second adaptor molecule may comprise a single-stranded portion. The single-stranded portion may form a hairpin or a stem-loop. Thus, the first adaptor molecule and/or the second adaptor molecule may comprise a loop portion. The single-stranded portion may comprise less than 10, 9, 8, 7, 6, 5, 4, 3, 2 nucleotides. Preferably, the single-stranded portion comprises 5 nucleotides.

The first adaptor molecule and/or the second adaptor molecule may comprise a double-stranded portion. The double-stranded portion may comprise less than 50, less than 45, less than 40, less than 35, less than 30, less than 25, less than 20, less than 15, or less than 10 base pairs. The double-stranded portion may comprise at least 5, at least 6, at least 7, at least 8, at least 9, at least 10, at least 11, at least 12, at least 13, at least 14, or at least 15 base pairs.

The first adaptor molecule and/or the second adaptor molecule may comprise a 5' phosphate. The 5' phosphate may facilitate ligation to the digested precursor dsDNA (which may comprise a 3'-OH group at first and/or second ends). The first adaptor molecule and/or the second adaptor molecule may comprise a 3'-OH. The 3'-OH may facilitate ligation to the digested precursor dsDNA (which may comprise a 5' phosphate at first and/or second ends).

The first adaptor molecule may comprise a portion that is complementary to the first end of the digested precursor dsDNA. The second adaptor molecule may comprise a portion that is complementary to the second end of the digested precursor dsDNA. The first adaptor molecule may comprise a portion that anneals to the first end of the digested precursor dsDNA. The second adaptor molecule may comprise a portion that anneals to the second end of digested precursor dsDNA. The first adaptor molecule may comprise a portion that is complementary and anneals to the first end of the digested precursor dsDNA. The second adaptor molecule may comprise a portion that is complementary and anneals to the second end of the digested precursor dsDNA.

The portion that is complementary or anneals to the first or second end of the digested precursor dsDNA may be a 5' overhang or a 3' overhang of the first and/or second adaptor molecule. The overhang of the first adaptor molecule may be complementary to the first end of the digested precursor dsDNA and/or the overhang of the second adaptor molecule may be complementary to the second end of the digested precursor dsDNA. The overhang of the first adaptor molecule may anneal to the first end of the digested precursor dsDNA and/or the overhang of the second adaptor molecule may anneal to the second end of the digested precursor dsDNA. The overhang of the first adaptor molecule may be complementary to and anneal to the first end of the digested precursor dsDNA and/or the overhang of the second adaptor molecule may be complementary to and anneal to the second end of the digested precursor dsDNA.

The first adaptor molecule may be appended to a first end of the digested precursor dsDNA and the second adaptor molecule may be appended to a second end of the digested precursor dsDNA. The appending of the first adaptor molecule and/or the second adaptor molecule may be performed by hybridization or ligation of the adaptor molecules to the ends of the digested precursor dsDNA. Thus, the first adaptor molecule may be hybridized to the first end of the digested precursor dsDNA. The second adaptor molecule may be hybridized to the second end of the digested precursor dsDNA. The first adaptor molecule may be ligated to the first end of the digested precursor dsDNA. The second adaptor molecule may be ligated to the second end of the digested precursor dsDNA. The appending of the first adaptor molecule and the second adaptor molecule may be performed by both hybridization and ligation of the adaptor molecules to the ends of the digested precursor dsDNA. Thus, the first adaptor molecule may be hybridized and ligated to the first end of the digested precursor dsDNA. The second adaptor molecule may be hybridized and ligated to the second end of the digested precursor dsDNA. The hybridization is based on complementarity of a portion of the first and/or second adaptor molecules to the first and/or second end of the digested precursor dsDNA.

The first adaptor molecule and/or the second adaptor molecule may not comprise a Type IIS endonuclease target sequence. The first adaptor molecule and/or the second adaptor molecule may not comprise Bbsl, Bsal, BsmBI, BspQl, BtgZl, Esp3I,Sapl, Aarl, Acc36I, AcIWI, Acul, Ajul, Alol, Alw26I, Alwl, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1I, Bse3DI, BseGI, BseMl, BseMII, BseNI, BseRl, BseXI, Bsgl, BsIFI, BsmAl, BsmFl, Bsml, Bso31I, BspCNI, BspMI, BspPl, BspQl, BspTNI, BsrDl, Bsrl, Bst6I, BstF5I, BstMAI, BstV1I, BstV2I, Bsul, BtgZl, BtsCl, Btsl-v2, BtsMutl, Bvel, Csel, CspCI, Eam1104I, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAIII, PaqCl, PciSI, Pctl, PleI, Ppsl, Psrl, Schl, SfaNl, Taqll, TspDTI and/or TspGWI target sequences.

The first adaptor molecule and/or the second adaptor molecule may comprise one or more locked nucleic acids (LNAs).

The first adaptor molecule and/or the second adaptor molecule may confer resistance to the nuclease digestion, such as exonuclease digestion (e.g. exonuclease III or VIII digestion).

The first adaptor molecule and/or the second adaptor molecule may comprise one or more protected nucleotides (i.e. nuclease-resistant nucleotides), such as phosphorothioated nucleotides. The protected nucleotides may be located in the single-stranded portion (e.g. hairpin portion) or the double-stranded portion. The protected nucleotides may be located in the overhang portion of the adaptor molecules.

The first and second adaptor molecules may comprise one or more phosphorothioated nucleotides, such that, once the adaptor molecules are appended (e.g. ligated) to the digested precursor dsDNA and the second strand of the partially protected dsDNA is digested by the exonuclease (or exonuclease mixture), the protected DNA is resistant to nuclease digestion or has improved or enhanced resistance to nuclease digestion. The protected DNA product may be resistant to 3'-end exonuclease digestion (e.g. by exonuclease III) and/or 5'-end exonuclease digestion (e.g. by exonuclease VIII).

The adaptor molecule may comprise a plurality of phosphorothioated nucleotides. For example, the adaptor molecules may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides in each strand. The first and/or second adaptor molecule may comprise protected nucleotides in the first strand and may not comprise protected nucleotides in the second strand.

The adaptor molecule may be a nucleic acid adaptor molecule. The adaptor molecule may be double-stranded. The adaptor molecule may comprise a portion that is double-stranded.

The first and/or second adaptor molecules may comprise at least 3, at least 4, at least 5, at least 6, at least 7, at least 8, at least 9, at least 10, at least 11, at least 12, at least 13, at least 14, at least 15, or at least 16 base pairs.

The adaptor molecule may comprise a plurality of phosphorothioated nucleotides at internal positions in each strand. For example, the adaptor molecules may comprise at least 1, at least 2, at least 3, at least 4, at least 5, at least 6, at least 7, at least 8, at least 9, at least 10, at least 11, at least 12, at least 13, at least 14, at least 15 or at least 16 phosphorothioated nucleotides at internal positions in each strand. Preferably, the adaptor molecule comprises at least 2 phosphorothioated nucleotides at internal positions in each strand.

The internal positions may not be located between the second and penultimate nucleotide of the adaptor molecule. The internal positions may be any position in the adaptor molecules other than the last nucleotide at the end of each strand.

The adaptor molecule may comprise at least 5%, at least 10%, at least 20%, at least 30%, at least 40%, at least 50%, at least 60%, at least 70%, at least 80%, at least 90%, at least 95%, or at least 100% of protected nucleotides.

Once the adaptor molecules are appended to the digested precursor dsDNA and the partially protected dsDNA is digested by the exonuclease (or exonuclease mixture), the protected DNA may comprise a protected nucleotide at the 5' end of the ssDNA cassette or 5' of the ssDNA cassette, and a nuclease-resistant nucleotide at the 3'-end of the ssDNA cassette or 3' of the ssDNA cassette.

The partially protected dsDNA may be generated from the precursor dsDNA by performing the steps described herein in a single reaction (i.e. as a single step) in a single contiguous aqueous volume. For example, the steps of contacting a precursor dsDNA with an endonuclease, digesting the precursor dsDNA with the endonuclease to generate a digested precursor dsDNA, contacting the digested precursor dsDNA with a ligase and first and second adaptor molecules, and ligating the first and second adaptor molecules to the digested precursor DNA thereby generating the partially protected dsDNA, may be performed in a single reaction (i.e. as a single step) in a single contiguous aqueous volume. Thus, the partially protected dsDNA may be generated by incubating the precursor dsDNA with the endonuclease, the ligase and first and second adaptor molecules in a single reaction (i.e. a single step) in a single contiguous aqueous volume. The single contiguous aqueous volume may be incubated in order to allow the required digestion and ligation.

The single contiguous aqueous volume may be incubated to generate the partially protected dsDNA by digesting the precursor dsDNA and ligating the first and second adaptor molecules to the digested precursor dsDNA.

The step of incubating the single contiguous aqueous volume may be performed under conditions that promote ligation of the first and second adaptor molecules to the digested precursor dsDNA to produce the partially protected dsDNA. The appending may be performed by creating a covalent link between the first and/or second adaptor molecule and the end(s) of the digested precursor dsDNA.

The step of incubating the single contiguous aqueous volume may be performed under conditions that promote digestion of the precursor dsDNA to produce the digested precursor dsDNA. The digestion of the precursor dsDNA to produce the digested precursor dsDNA may be performed at a first temperature of 1°C-100°C, 1°C -80°C, 5°C-70°C, 10°C-60°C, 15°C-55°C, 20°C-50°C, 25°C -45°C, 30°C-40°C, 35°C-39°C, 36°C-38°C, or at about 37°C. The digestion may be endonuclease digestion, preferably Type IIS endonuclease digestion.

The step of incubating the single contiguous aqueous volume may be performed under conditions that promote ligation of the digested precursor dsDNA to the first and second adaptor molecules. The ligation may be at least 5%, at least 10%, at least 15, at least 20%, at least 25%, at least 30%, at least 35%, at least 40, at least 45%, at least 50%, at least 55%, at least 60, at least 65, at least 70%, at least 75%, at least 80%, at least 82%, at least 85%, at least 90%, or at least 95% efficient. For example, at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% of the digested precursor dsDNA may be incorporated into partially protected dsDNA. Preferably, the ligation is at least 15% efficient.

The step of ligation of the digested precursor dsDNA to the first and second adaptor molecules may be performed at a second temperature of 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C.

The step of incubating the single contiguous aqueous volume may comprise incubating at a first temperature and then incubating at a second temperature. The first temperature may be 1°C-100°C, 1°C-80°C, 5°C-70°C, 10°C-60°C, 15°C-55°C, 20°C-50°C, 25°C-45°C, 30°C-40°C, 35°C-39°C, 36°C-38°C, or about 37°C. The second temperature may be 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C. Preferably, the first temperature is 35°C-39°C. Preferably, the second temperature is 14°C-18°C.

The step of incubating the single contiguous aqueous volume may be performed isothermally. The step of incubating the single contiguous aqueous volume may comprise incubating at a constant temperature. The constant temperature promotes simultaneous digestion of the double-stranded DNA molecule to produce the linear portion of the double-stranded DNA molecule and ligation of the linear double-stranded region to the first and second adaptor molecules. For example, the constant temperature may be 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, or 40°C. Preferably, the constant temperature is 30°C. The constant temperature is intended to mean that the temperature does not significantly change during the reaction. The constant temperature is intended to mean that the temperature variation during the step of incubating the single contiguous aqueous volume is less than 10°C, less than 9°C, less than 8°C, less than 7°C, less than 6°C, less than 5°C, less than 4°C, less than 3°C, less than 2°C, or less than 1°C. In a preferred embodiment the temperature during the step of incubating the single contiguous aqueous does not deviate by more than 5°C, preferably by not more than 3°C, even more preferably not more than 1°C. Thus, the constant temperature may be a temperature in a range of 20°C-30°C, 22°C-32°C, 24°C-34°C, 26°C-36°C, 28°C-38°C, 30°C-40°C, 22°C-28°C, 32°C-38°C, 25°C-35°C, 26°C-34°C, 27°C- 33°C, 27.5°C-32.5°C, 28°C-32°C, 28.5°C-31.5°C, 29°C-31°C, or 29.5°C-30.5°C. Preferably, the constant temperature is a temperature in a range of 27.5°C-32.5°C. Alternatively, the constant temperature may be a temperature in a range of 32°C-42°C, 33°C-41°C, 34°C-40°C, 35°C-39°C, 36°C-38°C. Preferably, the constant temperature is a temperature in a range of 34.5°C-39.5°C.

The step of incubating the single contiguous aqueous volume may comprise cycling between the first temperature and the second temperature. The step of incubating the single contiguous aqueous volume may comprise cycling between the first temperature and the second temperature at least 2, at least 3, at least 4, at least 5, at least 6, at least 7, at least 8, at least 9, at least 10, at least 15, at least 20, at least 25, at least 30, at least 35, at least 40, at least 45, at least 50, at least 55, at least 60, at least 65, at least 70, at least 80, at least 90, or at least 100 times, preferably at least 20 times. The step of incubating the single contiguous aqueous volume may comprise cycling between the first temperature and the second temperature less than 40, less than 35, less than 30 times, less than 29, less than 25 times. The step of incubating the single contiguous aqueous volume may comprise cycling between the first temperature and the second temperature 2-100, 5-80, 10-70, 20-60, or 30-60 times. The step of incubating the single contiguous aqueous volume may comprise cycling between the first temperature and the second temperature 2-20, 5-29, 61-100, or 65-80 times.

The DNA template may comprise at least one endonuclease target sequence. Thus, the DNA template may comprise at least one endonuclease target sequence. Preferably, the DNA template comprises at least two endonuclease target sequences. The endonuclease target sequences may be the same or different. Preferably, the at least one endonuclease target sequence is a restriction endonuclease target sequence. Different restriction endonuclease target sequences would be known to the skilled person. The endonuclease target sequence may be a Type IIS restriction endonuclease target sequence. For example, the restriction endonuclease target sequence may be a Bbsl, Bsal, BsmBI, BspQl, BtgZl, Esp3I,SapI, Aarl, Acc36I, AcIWI, Acul, Ajul, AloI, Alw26I, AlwI, Arsl, AsuHPI, Bael, Barl, Bbvl, Bccl, BceAl, Bcgl, BciVI, BcoDI, BfuAl, Bful, Bmrl, Bmsl, Bmul, Bpil, Bpml, BpuEl, BsaXI, Bse1I, Bse3DI, BseGI, BseMl, BseMll, BseNI, BseRl, BseXl, Bsgl, BsIFI, BsmAl, BsmFl, Bsml, Bso31I, BspCNI, BspMI, BspPl, BspQl, BspTNI, BsrDl, Bsrl, Bst6I, BstF5I, BstMAI, BstV1I, BstV2I, Bsul, BtgZl, BtsCl, Btsl-v2, BtsMutl, Bvel, Csel, CspCI, Eam1104I, Earl, Ecil, Eco31I, Eco57I, Esp3I, Faql, Faul, Fokl, Gsul, Hgal, Hphl, HpyAV, Lgul, Lmnl, Lsp1109I, Lwel, Mboll, Mlyl, Mmel, Mnll, Mva1269I, NmeAIII, PaqCl, PciSI, Pctl, PleI, Ppsl, Psrl, Schl, SfaNI, Taqll, TspDTI and/or TspGWI target sequence. The at least one endonuclease target sequence may be a native endonuclease sequence (i.e. an endonuclease sequence present in the template molecule). Alternatively, the at least one endonuclease target sequence may be introduced to the DNA template molecule prior to the production of the protected DNA.

The DNA template used in the methods described herein may be single-stranded or double-stranded. Preferably, the DNA template is double-stranded. The DNA template may be a natural circular DNA molecule. For example, the DNA template molecule may be (i) a plasmid, (ii) a minicircle, (iii) a cosmid, (iv) a bacterial artificial chromosome (BAC), or (v) a molecular inversion probe (MIP). The DNA template molecule may be an enzymatically produced circular DNA molecule. For example, the DNA template may be (i) a circular DNA molecule obtained from recombinase reaction, preferably Cre recombinase reaction, or (ii) a circular DNA molecule obtained from ligase reaction, preferably using the golden gate assembly. The DNA template may be an enzymatically produced covalently-closed linear DNA molecule. For example, the DNA template may be (i) a DNA molecule processed with TelN protelomerase; or (ii) a DNA molecule generated by ligation of the DNA ends with an adaptor. The DNA template molecule may comprise an element that is double-stranded and an element that is single-stranded. For example, the DNA template may comprise a double-stranded DNA and a single-stranded hairpin loop.

The DNA template may be linear. If the DNA template is linear, prior to amplification (e.g. rolling circle amplification), a DNA template may be circularized to produce a DNA template suitable for use in the methods described herein.

The digested precursor dsDNA may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the digested precursor dsDNA is at least 200 base pairs long.

The first end of the digested precursor dsDNA may be complementary to a portion of the first adaptor molecule. The second end of the digested precursor dsDNA may be complementary to a portion of the second adaptor molecule. The first end and/or the second end of the digested precursor dsDNA may be generated by endonuclease digestion.

The digested precursor dsDNA may comprise a 3'-OH group at first and/or second ends. The 3'-OH group may facilitate ligation to the first and/or second adaptor molecule(s) (which may comprise a 5' phosphate). The digested precursor dsDNA may comprise a 5' phosphate at first and/or second ends. The 5' phosphate may facilitate ligation to the first and/or second adaptor molecule(s) (which may comprise a 3'-OH group).

The digested precursor dsDNA may comprise an overhang. For example, the digested precursor dsDNA may comprise a 5' overhang or a 3' overhang. The digested precursor dsDNA may comprise a blunt end or blunt ends. The digested precursor dsDNA may comprise: a 5' overhang and a blunt end, two 5' overhangs, a 3' overhang and a blunt end, two 3' overhangs, or a 5' overhang and a 3' overhang. The overhang may have at least 3 nucleotides (preferably from 4 to 8 nucleotides). The overhang may be in the first or second strand of the digested precursor dsDNA.

### 4. Methods for producing an RNA product using an adaptor-ligated DNA product and a protected splint DNA

The methods provides herein may be combined to allow the more efficient production of an RNA product.

The invention provides a method for producing an RNA product, wherein the method comprises:
(I) amplifying a DNA template molecule, wherein the DNA template molecule comprises at least one endonuclease target sequence to generate an amplified DNA product, optionally wherein the DNA template molecule is amplified by any of the methods described herein;
(II) producing an adaptor-ligated DNA product as a template for in vitro transcription (IVT), wherein the adaptor-ligated DNA product is produced from the amplified DNA product, optionally wherein the adaptor-ligated DNA product is produced from the amplified DNA product using any of the methods described herein;
(III) producing a protected splint DNA, wherein the protected splint DNA is produced from the amplified DNA product, optionally wherein the protected splint DNA is produced from the amplified DNA product using any of the methods described herein (i.e. the amplified DNA product is a precursor dsDNA);
(IV) providing a first RNA molecule, a second RNA molecule and a third RNA molecule, wherein the first RNA molecule is a chemically synthesized RNA molecule, the second RNA molecule is produced by in vitro transcription (IVT) using the adaptor-ligated DNA product as template for the in vitro transcription (IVT), and the third RNA molecule is a chemically synthesized RNA molecule; and
(V) annealing the first, second and third RNA molecules to the protected splint DNA; and ligating the 3' end of the first RNA molecule to the 5' end of the second RNA molecule and ligating the 3' end of the second RNA molecule to the 5' end of the third RNA molecule.

The invention provides a method for producing an RNA product, wherein the method comprises:
(I) amplifying a DNA template molecule, wherein the DNA template molecule comprises at least one endonuclease target sequence to generate amplified DNA product, optionally wherein the DNA template molecule is amplified by any of the methods described herein;
(II) producing an adaptor-ligated DNA product as a template for in vitro transcription (IVT), wherein the adaptor-ligated DNA product is produced from the amplified DNA product, optionally wherein the adaptor-ligated DNA product is produced from the amplified DNA product using any of the methods described herein;
(III) producing a protected splint DNA, wherein the protected splint DNA is produced from the amplified DNA product, optionally wherein the protected splint DNA is produced from the amplified DNA product using any of the methods described herein (i.e. the amplified DNA product is a precursor dsDNA);
(IV) producing a second RNA by in vitro transcription (IVT) using the adaptor-ligated DNA product as a template for in vitro transcription (IVT);
(V) providing a first RNA molecule and a third RNA molecule, wherein the first RNA molecule is a chemically synthesized RNA molecule and the third RNA molecule is a chemically synthesized RNA molecule; and
(VI) annealing the first, second and third RNA molecules to the protected splint DNA; and ligating the 3' end of the first RNA molecule to the 5' end of the second RNA molecule and ligating the 3' end of the second RNA molecule to the 5' end of the third RNA molecule.

The step of amplifying a DNA template may be an *in vitro* or *in vivo* amplification. Preferably, the amplification is an *in vitro* amplification. For example, the amplification may be performed by rolling circle amplification (RCA), MALBAC method, traditional polymerase chain reaction (PCR), nucleic acid sequence-based amplification (NASBA), loop-mediated isothermal amplification (LAMP), helicase-dependent amplification (HDA), multiple displacement amplification (MDA) and recombinase polymerase amplification (RPA). Preferably, the amplification is rolling circle amplification (RCA).

Rolling circle amplification may be performed without any primers, or in the presence of a primer or multiple primers. For example, the primer may be a synthetic primer. The primers may be random primers. Rolling circle amplification may be performed in the presence of a primase. The primase may be TthPrimPol. Preferably, if the rolling circle amplification is performed without any primers, it is performed in the presence of a primase, such as TthPrimPol. Similarly, if a primer is used during amplification reaction, a primase is not used. The double-stranded DNA product may be generated by the rolling circle amplification in vitro under isothermal conditions using a suitable nucleic acid polymerase, such as Phi29 DNA polymerase.

The step of producing an adaptor-ligated DNA product as a template for in vitro transcription (IVT) may be performed using first and second adaptor molecules. The first and second adaptor molecules may be any of the first and second adaptor molecules described herein in relation to the production of an adaptor-ligated DNA product.

The step of producing a protected splint DNA may be performed using first and second adaptor molecules. The first and second adaptor molecules may be any of the first and second adaptor molecules described herein in relation to the production of a protected splint DNA.

The first and second adaptor molecules used to produce an adaptor-ligated DNA product may be different to the first and second adaptor molecules used to produce a protected splint DNA. Thus, the first and second adaptor molecules used to produce a protected splint DNA may also be referred to herein as third and fourth adaptor molecules.

The method may further comprise digesting the splint DNA using DNase. The DNase may be DNase I and/or Duplex DNase.

The method may further comprise digesting the unligated RNA molecules using one or more RNA exonucleases. The RNA exonuclease(s) may be a 5'-3' RNA exonuclease and/or a 3'-5' RNA exonuclease. The one or more RNA exonucleases may be selected from XRN-I (5' to 3'), Terminator 5'-Phosphate-Dependent Exonuclease (5' to 3'), Exonuclease T (3' to 5'), Exolll (3' to 5') and/or RNase R (3' to 5').

### 5. Use of nuclease-resistant nucleotides (or protected nucleotides) in the methods and products

The RNA molecules and/or the DNA molecules (e.g. the DNA splint) may comprise one or more nuclease-resistant (or protected nucleotides). Preferably, the RNA molecules and/or the DNA molecules (e.g. the DNA splint) may each comprise two or more nuclease-resistant (or protected nucleotides). As used herein the term "nuclease-resistant nucleotide" or "protected nucleotide" is intended to encompass any type of nucleotide that provides or enhances resistance to nuclease digestion (especially exonuclease digestion).

The nuclease-resistant nucleotide(s) (i.e. a nucleotide(s) resistant to exonuclease digestion) may be a phosphorothioated nucleotide (PS). As used herein, the term "phosphorothioated nucleotide" refers to a nucleotide that has an altered phosphate backbone, wherein the sugar moieties are linked by a phosphorothioate bond. In the phosphate backbone of an oligonucleotide sequence, the phosphorothioate bond contains a sulphur atom as a substitute for a non-bridging oxygen atom. This modification renders the internucleotide linkage resistant to nuclease degradation.

The phosphorothioated nucleotide(s) may comprise α-S-dATP (i.e. 2'-deoxyadenosine-5'-(α-thio)-triphosphate), α-S-dCTP (i.e. 2'-deoxycytidine-5'-(α-thio)-triphosphate), α-S-dGTP (i.e. 2'-deoxyguanosine-5'-(α-thio)-triphosphate), α-S-dTTP (i.e. 2'-deoxythymidine-5'-(α-thio)-triphosphate), α-S-dUTP (i.e. 2'-deoxyuridine-5'-(α-thio)-triphosphate), and/or uridine 2', 3'-cyclophosphorothioate.

The phosphorothioated nucleotide(s) may be Sp-isomers, Rp-isomers or a mixture of both Sp- and Rp-isomers.

The nuclease-resistant nucleotide(s) (i.e. nucleotide(s) resistant to exonuclease digestion) may be 2'-O-methyl (2' O-Me) nucleotides or 2'-O-methoxyethyl (MOE) nucleotides.

The nuclease-resistant nucleotide(s) may be 2' O-Me nucleotides of at least one type, or at least two, at least three or at least four types. For example, the 2' O-Me nucleotide(s) may comprise one or more of the following nucleosides: 2'-O-methyl adenosine, 2'-O-methyl cytidine, 2'-O-methyl guanosine, 2'-O-methyl thymidine and/or 2'-O-methyl uridine.

The nuclease-resistant nucleotide(s) may be MOE nucleotide(s) of at least one type, or at least two, at least three or at least four types. For example, the MOE nucleotide(s) may comprise one or more of the following nucleosides: 2'-O-methoxy-ethyl adenosine, 2'-O-methoxy-ethyl cytidine, 2'-O-methoxy-ethyl guanosine, 2'-O-methoxy-ethyl thymidine and/or 2'-O-methoxy-ethyl uridine.

The nuclease-resistant nucleotide(s) may comprise 5-Methylcytidine(s).

The nuclease-resistant nucleotide(s) may be locked nucleic acid(s) (LNA).

The nuclease-resistant nucleotide(s) may comprise pseudouridine(s) and/or N1-Methyl-Pseudouridine(s).

### 6. RNA products

The invention provides an RNA product as described herein.

The RNA product may be a linear RNA product.

The RNA product may comprise a long RNA, a long non-coding RNA (IncRNA), a non-coding RNA (ncRNA), a guide RNA (gRNA), a single guide RNA (sgRNA), a prime editing guide RNA (pegRNA) or an mRNA (e.g. an mRNA encoding a pathogen-specific antigen or an mRNA encoding a neoantigen). The gRNA may comprise an aptamer sequence (e.g. MS2 aptamer sequence).

The RNA product may comprise an RNA cassette. The RNA cassette may comprise a long RNA, a long non-coding RNA (IncRNA), a non-coding RNA (ncRNA), a guide RNA (gRNA), a prime editing guide RNA (pegRNA) or an mRNA (e.g. an mRNA encoding a pathogen-specific antigen or an mRNA encoding a neoantigen). The gRNA may comprise an aptamer sequence (e.g. MS2 aptamer sequence).

The RNA product may be at least 50 nucleotides, at least 75 nucleotides, at least 100 nucleotides, at least 105 nucleotides, at least 110 nucleotides, at least 115 nucleotides, at least 120 nucleotides, at least 125 nucleotides, at least 135 nucleotides, at least 145 nucleotides, at least 150 nucleotides, at least 155 nucleotides, at least 160 nucleotides, at least 165 nucleotides, at least 170 nucleotides, at least 175 nucleotides, at least 180 nucleotides, at least 185 nucleotides, at least 190 nucleotides, at least 195 nucleotides, at least 200 nucleotides, at least 250 nucleotides, at least 300 nucleotides, at least 400 nucleotides, at least 500 nucleotides, at least 750 nucleotides, at least 1000 nucleotides, at least 1500 nucleotides, at least 2000 nucleotides, at least 3000 nucleotides, at least 4000 nucleotides, at least 5000 nucleotides, at least 6000 nucleotides, at least 7000 nucleotides, at least 8000 nucleotides or at least 9000 nucleotides. Preferably, the RNA product is at least 135 nucleotides.

The RNA product may be a protected linear RNA. The RNA product may comprise two or more nuclease resistant nucleotides (i.e. nucleotides resistant to exonuclease digestion). The RNA product may be resistant to 5'-3' exonuclease digestion and/or 3'-5' exonuclease digestion. The RNA product may comprise at least 1 nuclease resistant nucleotide 5' of the RNA cassette and at least 1 nuclease resistant nucleotide 3' of the RNA cassette. The RNA product may comprise at least 2 nuclease resistant nucleotides 5' of the RNA cassette and at least 2 nuclease resistant nucleotides 3' of the RNA cassette. The RNA product may comprise at least 3 nuclease resistant nucleotides 5' of the RNA cassette and at least 3 nuclease resistant nucleotides 3' of the RNA cassette. The RNA product may comprise at least 4 nuclease resistant nucleotides 5' of the RNA cassette and at least 4 nuclease resistant nucleotides 3' of the RNA cassette. The RNA product may comprise at least 5 nuclease resistant nucleotides 5' of the RNA cassette and at least 5 nuclease resistant nucleotides 3' of the RNA cassette. Preferably, the RNA product comprises at least 2 nuclease resistant nucleotides 5' of the RNA cassette and at least 2 nuclease resistant nucleotides 3' of the RNA cassette.

The invention provides an RNA product (e.g. a guide RNA or a pegRNA) produced or obtainable by the methods for producing an RNA product as described herein.

### BRIEF DESCRIPTION OF THE DRAWINGS

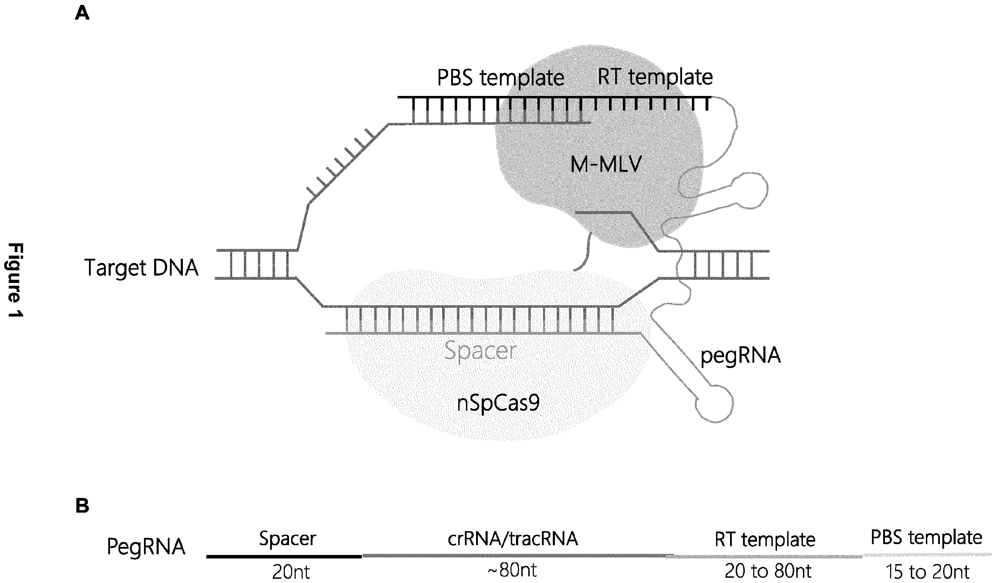

**FIG. 1** (A) Schematic illustrating the components of a prime editing (PE) ribonucleoprotein complex (RNP) and a target DNA. The PE RNP includes a prime editing guide RNA (pegRNA and a fusion protein of Moloney Murine Leukemia Virus Reverse Transcriptase (M-MLV) and Streptococcus pyogenes Cas9 nickase (nSpCas9). The positions of the Primer Binding Site (PBS) template, Reverse Transcription (RT) template and Spacer on the pegRNA are shown. (B) Schematic of a pegRNA illustrating from 5' to 3' the following sequence elements (and their sizes): Spacer, CRISPR RNA (crRNA)/trans-activating CRISPR RNA (tracRNA), Reverse Transcription (RT) template and Primer Binding Site (PBS) template.

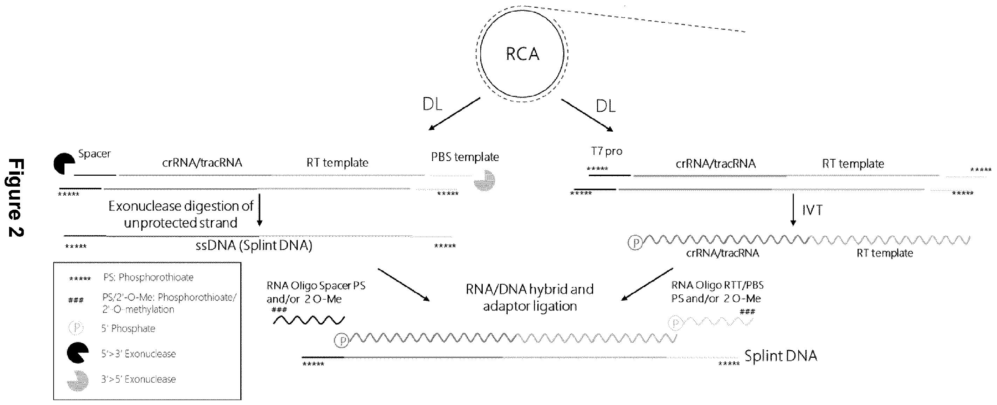

**FIG. 2** Schematic illustrating a method for producing a pegRNA. In the method, rolling circle amplification (RCA) is used to produce a concatemer comprising sequences of a pegRNA. The concatemer is then added to a digestion and ligation reaction (DL) with first and second adaptor molecules; in each case, the first adaptor molecule is ligated to a first end of a digested monomer of the concatemer and the second adaptor molecule is ligated to a second of a digested monomer of the concatemer. A first set of adaptor molecules are used in the DL reaction on the left side of the figure to produce a double-stranded DNA molecule with a lower strand with protected nucleotides at either end. Exonucleases are then used to digest the upper strand to produce a single-stranded DNA (ssDNA) that will serve as the splint DNA. A second set of adaptor molecules are used in the DL reaction on the right side of the figure to produce a double-stranded DNA molecule with a T7 promoter (T7 pro). This double-stranded DNA molecule is then used as a template for in vitro transcription (IVT) to produce an RNA comprising crRNA/tracRNA and RT template sequences (this is an example of a second RNA molecule). In the final step, a first RNA molecule (RNA Oligo Spacer PS and/or 2 O-Me) and a third RNA molecule (RNA Oligo RTT/PBS PS and/or 2 O-Me) are ligated to either end of the second RNA molecule by splint ligation using the splint DNA. In this example, the first RNA molecule and the third RNA molecule have been chemically synthesized.

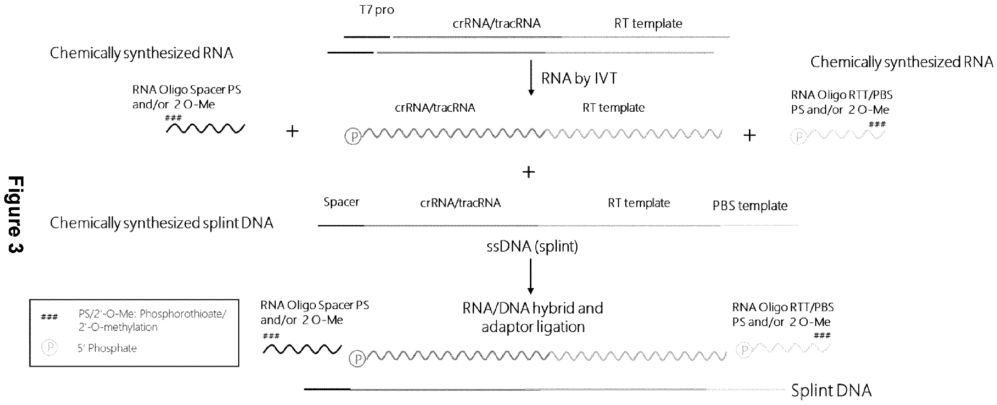

**FIG. 3** Schematic illustrating a method for producing a pegRNA. In the method, a double-stranded DNA molecule with a T7 promoter (T7 pro) is used as a template for in vitro transcription (IVT) to produce an RNA comprising crRNA/tracRNA and RT template sequences (this is an example of a second RNA molecule). In addition, a first RNA molecule (RNA Oligo Spacer PS and/or 2 O-Me), a third RNA molecule (RNA Oligo RTT/PBS PS and/or 2 O-Me) and a splint DNA are chemically synthesized. In the final step, the first RNA molecule and the third RNA molecule are ligated to either end of the second RNA molecule by splint ligation using the splint DNA.

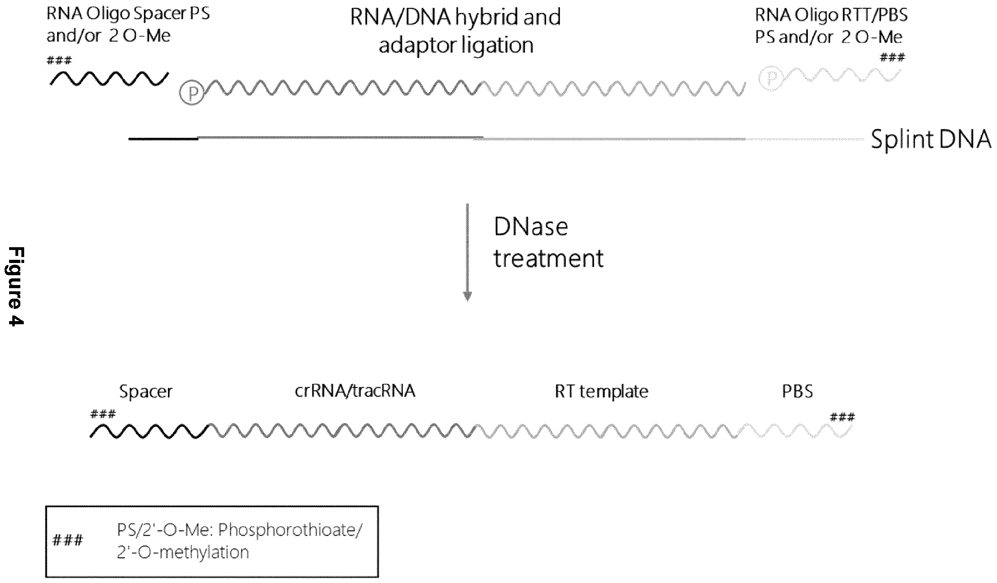

**FIG. 4** Schematic illustrating a splint ligation reaction (formed of an RNA/RNA hybrid) followed by DNase treatment to remove the DNA strand leaving a single-stranded pegRNA.

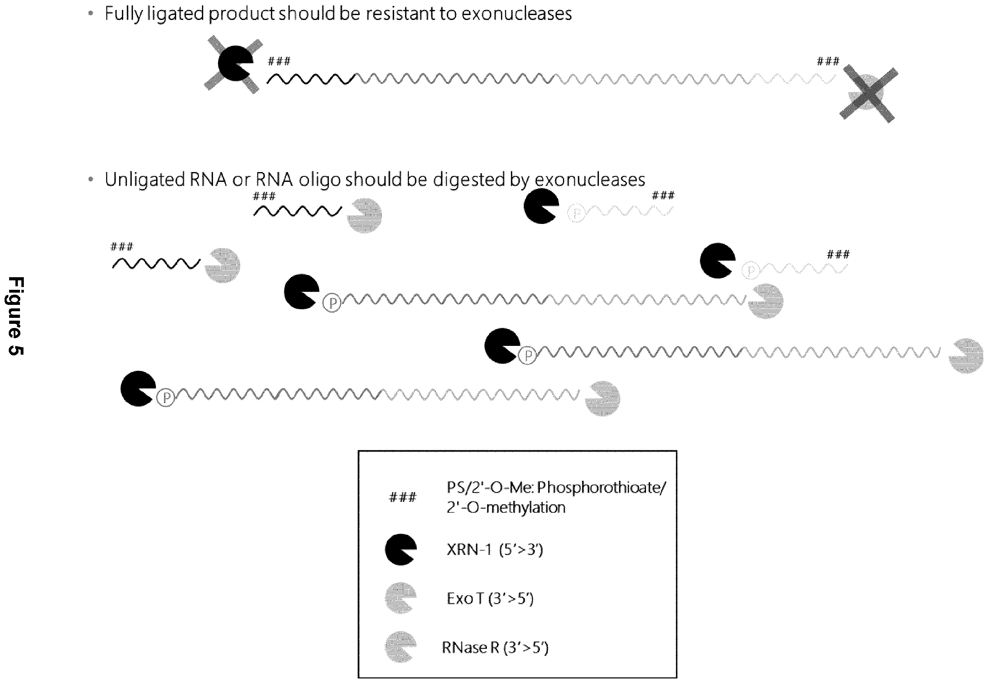

**FIG. 5** Schematic illustrating how all unligated products are degraded by exonuclease because they are not protected on both ends. Only the fully ligated product formed of three RNA molecules (as illustrated in FIG. 2 and 3) has protected nucleotides at both ends and is resistant to degradation by exonucleases.

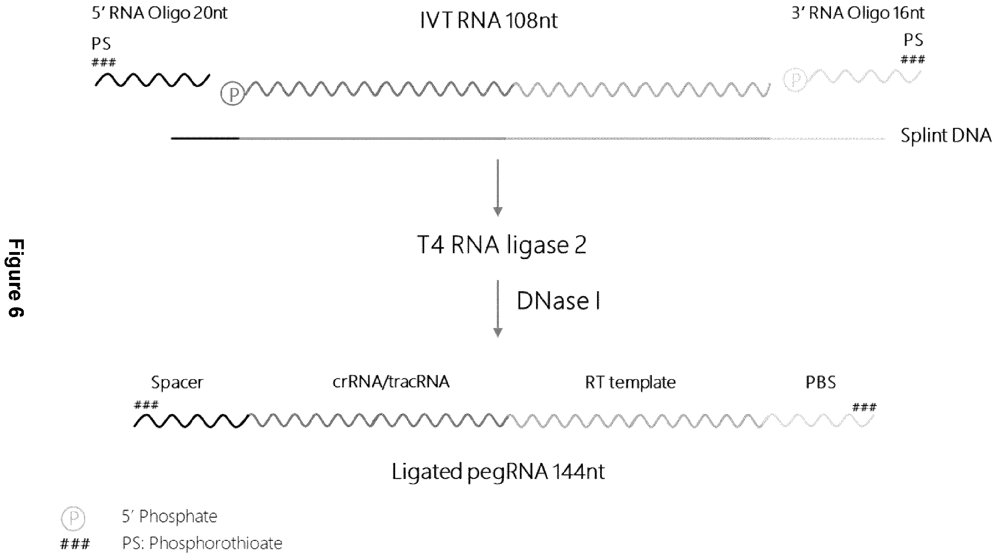

**FIG. 6** Schematic of the three RNA molecule splint ligation strategy for generating pegRNAs. The first RNA molecule (5'RNA Oligo 20nt; SEQ ID NO: 1) is a chemically synthesized 20 nucleotide phosphorothioated (PS) RNA encoding a spacer sequence. The second RNA molecule (IVT RNA 108nt; SEQ ID NO: 2) is 108 nucleotide RNA synthesized by in vitro transcription (IVT) encoding a scaffold region and a first portion of a Reverse transcription template (RTT). The third RNA molecule (3'RNA Oligo 16nt; SEQ ID NO: 3) is a chemically synthesized 16 nucleotide phosphorothioated (PS) RNA encoding a second portion of the RTT and a primer binding site (PBS). The figure shows the three RNA molecules annealed to a splint DNA. Ligation is performed using T4 RNA ligase 2 followed by DNase I treatment to degrade the splint DNA leaving the 144 nucleotide single-stranded pegRNA product (Ligated pegRNA 144nt).

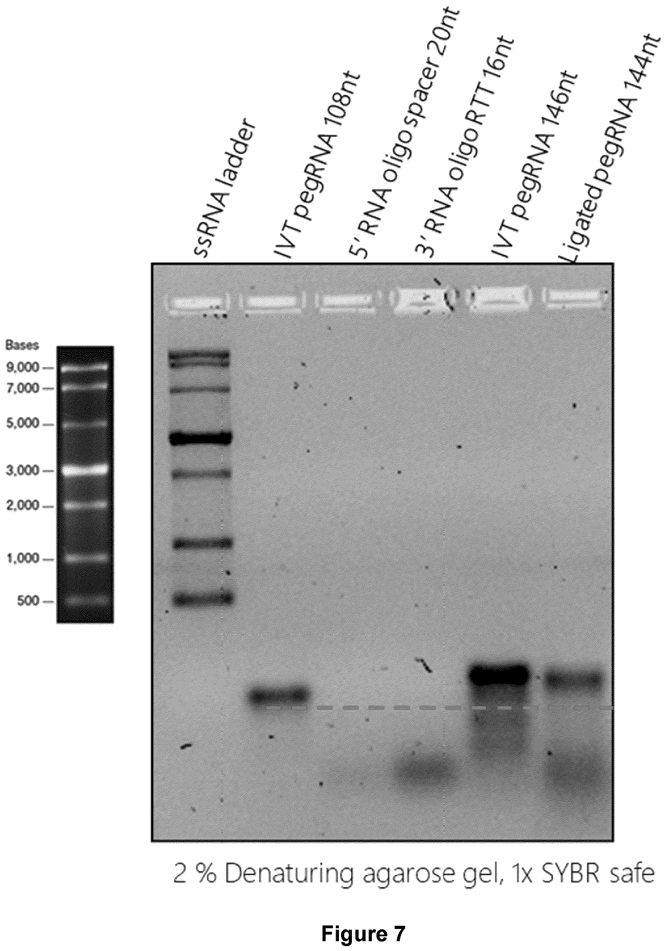

**FIG. 7** 2% denaturing agarose gel of three molecule ligation products: IVT pegRNA 108nt indicates an unligated second RNA molecule (i.e. a 108 nucleotide synthesized by in vitro transcription (IVT) encoding a scaffold region and a first portion of a Reverse transcription template (RTT)); 5'RNA oligo spacer 20nt indicates an unligated first RNA molecule (i.e. a chemically synthesized 20 nucleotide phosphorothioated (PS) RNA encoding a spacer sequence); 3'RNA oligo RTT 16nt indicates an unligated third RNA molecule (i.e. a chemically synthesized 16 nucleotide phosphorothioated (PS) RNA encoding a second portion of the RTT and a primer binding site (PBS)); IVT pegRNA 146nt indicates the full-length IVT synthesized size-control RNA; and Ligated pegRNA 144nt indicates full-length pegRNA (144 nucleotides) formed of first, second and third RNA molecules (as illustrated in FIG.6). On the left side of the figure is an annotated version of the ssRNA ladder shown in the main image.

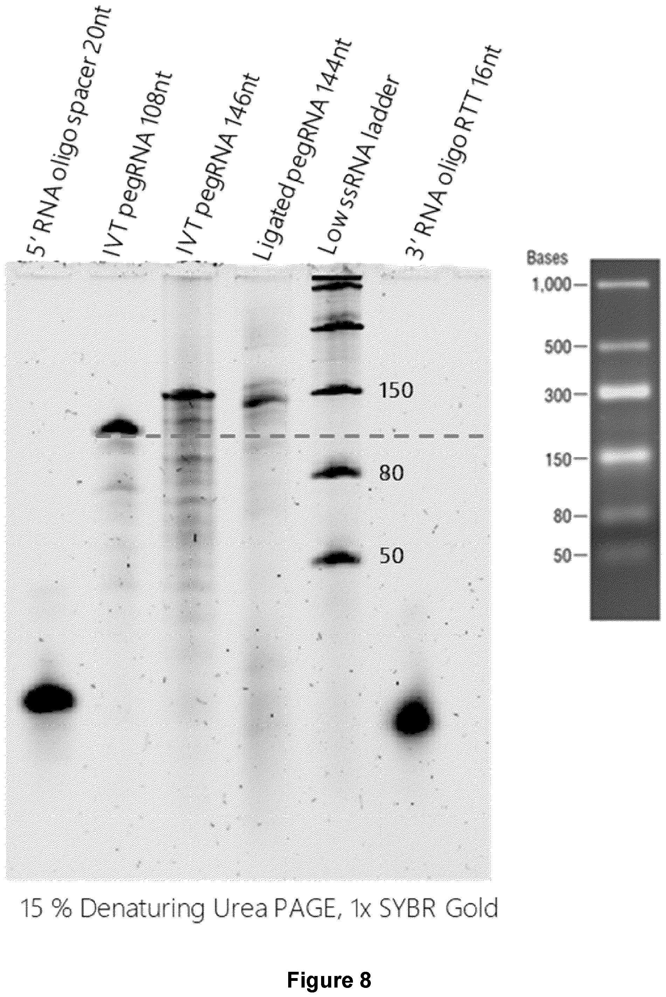

**FIG. 8** 15% denaturing Urea PAGE of three RNA molecule ligation products: 5'RNA oligo spacer 20nt indicates an unligated first RNA molecule (i.e. a chemically synthesized 20 nucleotide phosphorothioated (PS) RNA encoding a spacer sequence); IVT pegRNA 108 nt indicates an unligated second RNA molecule (i.e. a 108 nucleotide synthesized by in vitro transcription (IVT) encoding a scaffold region and a first portion of a Reverse transcription template (RTT)); IVT pegRNA 146nt indicates the full-length IVT synthesized size-control RNA; Ligated pegRNA 144 nt indicates full-length pegRNA (144 nucleotides) formed of first, second and third RNA molecules (as illustrated in FIG.6); Low ssRNA ladder; and 3'RNA oligo RTT 16nt indicates an unligated third RNA molecule (i.e. a chemically synthesized 16 nucleotide phosphorothioated (PS) RNA encoding a second portion of the RTT and a primer binding site (PBS)). On the right side of the figure is an annotated version of the Low ssRNA ladder shown in the main image.

### EXAMPLES

Nucleic acid synthesis: the following nucleic acids were chemically synthesized by Integrated DNA Technologies (IDT):
- an RNA oligonucleotide of 20 nucleotides encoding a spacer sequence (herein a first RNA molecule) and including phosphorothioated (PS) nucleotides at the 5' end (SEQ ID NO: 1);
- an RNA oligonucleotide of 16 nucleotide encoding a portion of the RTT and a primer binding site (PBS) (herein a third RNA molecule) and including phosphorothioated (PS) nucleotides at the 3' end (SEQ ID NO: 3);
- a single stranded splint DNA (SEQ ID NO: 4); and
- a double stranded DNA to serve as a template (dsDNA template) for in vitro transcription (IVT) (SEQ ID NO: 5).

The sequences of these nucleic acids are provided in Table 1.

In vitro transcription (IVT) of the pegRNA scaffold region: IVT was performed using T7 RNA polymerase in a reaction mixture containing 5× T7 RNA Polymerase Reaction Buffer and T7 RNA polymerase (T7 Flashscribe kit, Cellscript), 10 mM DTT, pyrophosphatase and 2.5 µg dsDNA template in a total volume of 50 µl. Nucleoside triphosphates (GTP, ATP, CTP, UTP) were supplemented to a final concentration of 10mM and the reaction mixture was incubated at 37 °C for 120 min. The dsDNA template was then enzymatically digested by adding 2 ul of RNase-Free DNase (T7 Flashscribe kit, Cellscript) at 37 °C for 15 min. The IVT RNA was purified with sodium acetate or ammonium acetate precipitation solution (Invitrogen). The IVT RNA (herein a second RNA molecule) is a 108 nucleotide RNA encoding a scaffold region and a first portion of a Reverse transcription template (RTT).

Enzymatic treatment and ligation: The IVT RNA was dephosphorylated using 5' Pyrophosphohydrolase (RPPH) (NEB) to leave a single 5'-phosphate for the subsequent ligation reaction. For RNA ligation, 250 pmol each of the three RNA molecules, and an equimolar amount of the single stranded splint DNA, were added to 1× annealing buffer (60mM NaCl, 6mM Hepes pH 7.5, 0.2mM MgCl2) in a total volume of 100 µl. This mixture was incubated for 2 min at 90 °C and then gradually cooled to room temperature. Subsequently, ligation was performed with 2 µl of 10× T4 RNA Ligase 2 buffer, T4 RNA Ligase 2 (NEB) as per manufacturer's instructions, followed by incubation at 25 °C for 1 h. The splint DNA was digested with RNase-Free DNase (Cellscript) for 20 min at 37 °C. The ligation product was purified with sodium acetate precipitation solution and resuspended in nuclease free water.

Samples of the purified ligation product were analysed using 2% denaturing agarose gel electrophoresis (Figure 7) and 15% denaturing urea polyacrylamide gel electrophoresis (PAGE) (Figure 8). Figures 7 and 8 show that a high purity full length ligated pegRNA of 144 nucleotides was produced (see Ligated pegRNA 144 nt in Figures 7 and 8). The absence of unligated RNA components confirms a highly efficient splint ligation of the three RNA molecules.

**Table 1**

| **SEQ ID NO:** | **Sequence description** | **Sequence 5'-3'** |
|---|---|---|
| 1 | First RNA molecule (5'RNA oligo spacer 20nt) | rC*rU*rC*rGrUrGrArCrCrArCrCrCrUrGrArGrCrCrA |
| 2 | Second RNA molecule (IVT pegRNA 108nt) | /5phos/rGrGrUrUrUrUrArGrArGrCrUrArGrArArArUrArG rCrArArGrUrUrArArArArUrArArGrGrCrUrArGrU rCrCrGrUrUrArUrCrArArCrUrUrGrArArArArArG rUrGrGrCrArCrCrGrArGrUrCrGrGrUrGrCrGrGrU rArGrCrGrGrCrGrArArGrCrArCrUrGrCrArCrG rCrCrGrUrArGrGrU |
| 3 | Third RNA molecule (3'RNA oligo RTT 16nt) | /5Phos/rGrArArGrGrUrGrGrUrCrArCrG*rA*rG*rG |
| 4 | Splint DNA (complementary to ligated 4bb_pegRNA 144 nt) | CCTCGTGACCACCTTCACCTACGGCGTGCAGTGCTTCGCCCGCTACCGCA CCGACTCGGTGCCACTTTTTCAAGTTGATAACGGACTAGCCTTATTTTAA CTTGCTATTTCTAGCTCTAAAACCTGGCTCAGGGTGGTCACGAG |
| 5 | dsDNA template (133nt) for IVT of SEQ ID NO: 2 (non-template strand shown) | CCCTCGAGTAATACGACTCACTATAGGTTTTAGAGCTAGAAATAGCAAGT TAAAATAAGGCTAGTCCGTTATCAACTTGAAAAAGTGGCACCGAGTCGGT GCGGTAGCGGGCGAAGCACTGCACGCCGTAGGT |
| 6 | Full length ligated pegRNA (Ligated 4bb_pegRNA 144nt) | rC*rU*rC*rGrUrGrArCrCrArCrCrCrUrGrArGrCrCrA rGrGrUrUrUrUrArGrArGrCrUrArGrArArArUrArG rCrArArGrUrUrArArArArUrArArGrGrCrUrArGrU rCrCrGrUrUrArUrCrArArCrUrUrGrArArArArArG rUrGrGrCrArCrCrGrArGrUrCrGrGrUrGrCrGrGrU rArGrCrGrGrGrCrGrArArGrCrArCrUrGrCrArCrG rCrCrGrUrArGrGrUrGrArArGrGrUrGrGrUrCrArC rG*rA*rG*rG |
| 7 | dsDNA template (171 nt) for IVT pegRNA 146nt (non-template strand shown) | CCCTCGAGTAATACGACTCACTATAGGCTCGTGACCACCCTGAGCCAGGT TTTAGAGCTAGAAATAGCAAGTTAAAATAAGGCTAGTCCGTTATCAACTT GAAAAAGTGGCACCGAGTCGGTGCGGTAGCGGGCGAAGCACTGCACGCCG TAGGTGAAGGTGGTCACGAGG |
| 8 | IVT pegRNA 146nt | /5TriPhos/rGrGrCrUrCrGrUrGrArCrCrArCrCrCrUrGrArGrC rCrArGrGrUrUrUrUrArGrArGrCrUrArGrArArArU rArGrCrArArGrUrUrArArArArUrArArGrGrCrUrA rGrUrCrCrGrUrUrArUrCrArArCrUrUrGrArArArA rArGrUrGrGrCrArCrCrGrArGrUrCrGrGrUrGrCrG rGrUrArGrCrGrGrGrCrGrArArGrCrArCrUrGrCrA rCrGrCrCrGrUrArGrGrUrGrArArGrGrUrGrGrUrC rArCrGrArGrG |

| | | |
|---|---|---|
| * indicates a phosphorothioated linkage between two nucleotides. For example, "G*G" means that the phosphorothioated linkage is between the two G nucleotides. /5phos/ denotes a 5'-phosphate (i.e. a 5' GMP). /5TriPhos/ denotes 5'-triphosphate (i.e. a 5' GTP) "r" preceding A, C, G or U denotes a ribonucleotide. | | |

## Claims

1. A method for producing a guide RNA, wherein the method comprises:
a) providing a first RNA molecule, a second RNA molecule, and a third RNA molecule, wherein the first RNA molecule comprises a nuclease-resistant nucleotide and the third RNA molecule comprises a nuclease-resistant nucleotide; and
b) ligating the 3' end of the first RNA molecule to the 5' end of the second RNA molecule and ligating the 3' end of the second RNA molecule to the 5' end of the third RNA molecule, wherein the ligation is performed by splint ligation;
wherein the guide RNA comprises, in the 5'-3' direction, a spacer sequence and a scaffold region; and
wherein the first RNA molecule is a chemically synthesized RNA molecule, the second RNA molecule is an in vitro transcription (IVT) produced RNA molecule, and the third RNA molecule is a chemically synthesized RNA molecule.

2. The method of claim 1, wherein the first RNA comprises at least a portion of the spacer sequence and the second RNA molecule comprises at least a portion of the scaffold region.

3. The method of claim 1 or claim 2, wherein the guide RNA is a prime editing guide RNA (pegRNA) and wherein the pegRNA comprises, in the 5'-3' direction, a spacer sequence, a scaffold region, a reverse transcription template (RTT) and a primer binding site (PBS).

4. The method of claim 3, wherein either:
a) the second RNA molecule comprises the reverse transcription template (RTT);
b) the third RNA molecule comprises the reverse transcription template (RTT); or
c) the second RNA molecule comprises a portion of the reverse transcription template (RTT) and the third RNA molecule comprises a portion of the reverse transcription template (RTT).

5. The method of claim 3 or claim 4, wherein the third RNA molecule comprises the PBS.

6. The method of any one of claims 1-5, wherein step (a) comprises: producing the first RNA molecule and the third RNA molecule by chemical synthesis; and producing the second RNA molecule by in vitro transcription (IVT), optionally wherein the chemical synthesis comprises solid-phase synthesis.

7. The method of any one of claims 1-6, wherein the second RNA molecule has a 5'-terminal GTP and step (a) comprises treating the second RNA molecule with a phosphatase to cleave phosphate groups from the 5'-terminal GTP to produce a second RNA molecule with a 5'-terminal GMP.

8. The method of any one of claims 1-7, wherein step (b) comprises: annealing the first, second and third RNA molecules to a splint DNA; and ligating the 3' end of the first RNA molecule to the 5' end of the second RNA molecule and ligating the 3' end of the second RNA molecule to the 5' end of the third RNA molecule; optionally wherein the splint DNA is a single splint DNA molecule.

9. The method of claim 8, wherein the splint DNA is a protected DNA comprising a single-stranded DNA (ssDNA) cassette, wherein the protected DNA comprises x nuclease-resistant nucleotides at the 5' end of the ssDNA cassette or 5' of the ssDNA cassette, and y nuclease-resistant nucleotides at the 3'-end of the ssDNA cassette or 3' of the ssDNA cassette, wherein x is at least 1 and y is at least 1.

10. The method of claim 8, wherein the splint DNA is a protected DNA comprising a single-stranded DNA (ssDNA) cassette, and wherein the method comprises:
(a) providing a partially protected double-stranded DNA (dsDNA) comprising a first strand and a second strand, wherein the first strand of the partially protected dsDNA comprises a cassette, x nuclease-resistant nucleotides at the 5' end of the cassette or 5' of the cassette, and y nuclease-resistant nucleotides at the 3'-end of the cassette or 3' of the cassette, wherein x is at least 1 and y is at least 1; and
(b) digesting the second strand of the partially protected dsDNA with an exonuclease thereby generating the protected DNA.

11. The method of any one of claims 8-10, wherein the splint DNA comprises a sequence comprising regions complementary to the scaffold region and spacer sequence.

12. The method of any one of claims 8-11, wherein the method further comprises (c) digesting the splint DNA using DNase.

13. The method of any one of claims 1-12, wherein step (b) is performed using T4 RNA ligase.

14. The method of any one of claims 1-13, wherein the first RNA molecule comprises a nuclease-resistant nucleotide 5' of the spacer sequence and/or wherein the third RNA molecule comprises a nuclease-resistant nucleotide.

15. A guide RNA for use in a method of treating a disease, wherein the guide RNA is produced according to the method of any one of claims 1-14 and wherein the guide RNA is used to edit a target gene by gene editing and thereby treat the disease.
